(19) Europäisches Patentamt
European Patent Office
Office européen des brevets

(11) Veröffentlichungsnummer: **0 306 649 B1**

# (12) EUROPÄISCHE PATENTSCHRIFT

(45) Veröffentlichungstag der Patentschrift: **04.03.92**

(51) Int. Cl.5: **C07C 69/732**, C07C 59/48, C07C 67/00, C07C 51/00, C07C 33/28, C07C 69/65, C07C 69/738, C07D 309/30

(21) Anmeldenummer: **88110833.6**

(22) Anmeldetag: **07.07.88**

## (54) Neue 3,5-Dihydroxycarbonsäuren und deren Derivate, Verfahren zu ihrer Herstellung, ihre Verwendung als Arzneimittel, pharmazeutische Präparate und Zwischenprodukte.

(30) Priorität: **10.07.87 DE 3722807**

(43) Veröffentlichungstag der Anmeldung:
**15.03.89 Patentblatt 89/11**

(45) Bekanntmachung des Hinweises auf die Patenterteilung:
**04.03.92 Patentblatt 92/10**

(84) Benannte Vertragsstaaten:
**AT BE CH DE ES FR GB GR IT LI LU NL SE**

(56) Entgegenhaltungen:
**EP-A- 0 011 928**
**EP-A- 0 014 846**
**EP-A- 0 024 648**
**EP-A- 0 095 875**
**EP-A- 0 119 067**
**EP-A- 0 207 456**
**EP-A- 0 211 205**
**EP-A- 0 234 496**
**DE-A- 2 212 948**
**DE-A- 2 300 328**
**DE-B- 1 145 161**
**DE-B- 1 193 490**
**GB-A- 2 162 179**
**US-A- 3 919 304**
**US-A- 4 335 054**
**US-A- 4 459 422**
**US-A- 4 567 289**

(73) Patentinhaber: **HOECHST AKTIENGESELLSCHAFT**
**Postfach 80 03 20**
**W-6230 Frankfurt am Main 80(DE)**

(72) Erfinder: **Wess, Günther, Dr.**
**Langenselbolder Weg 35**
**W-6455 Erlensee(DE)**
Erfinder: **Bartmann, Wilhelm, Prof., Dr.**
**Am Dachsbau 5**
**W-6232 Bad Soden am Taunus(DE)**
Erfinder: **Beck, Gerhard, Dr.**
**Gustav-Freytag-Strasse 24**
**W-6000 Frankfurt am Main(DE)**
Erfinder: **Granzer,Ernold, Dr. Dr.**
**Falkensteiner Strasse 24**
**W-6233 Kelkheim (Taunus)(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 306 649 B1

## Beschreibung

Das Enzym 3-Hydroxy-3-methylglutaryl-Coenzym-A-Reduktase (HMG-CoA-Reduktase) katalysiert die Bildung von Mevalonsäure aus 3-Hydroxy-3-methylglutaryl-Coenzym A (HMG-CoA). Diese Reaktion spielt eine zentrale Rolle bei der Biosynthese des Cholesterins. Derivate der 3-Hydroxy-3-methyl-glutarsäure (HMG) und der Mevalonsäure sind als Hemmer der Cholesterinbiosynthese beschrieben worden (M.R. Boots et al., J. Pharm. Sci. 69, 306 (1980), F.M. Singer et al., Proc.Soc. Exper. Biol.Med. 102, 270 (1959), H. Feres. Tetrahedron Lett. 24, 3769 (1983)). 3-Hydroxy-3-methyl-glutarsäure selbst zeigt an der Ratte und in Humanversuch signifikante cholesterinsenkende Wirkung (Z. Beg, Experientia 23, 380 (1967), ibid 24, 15 (1968), P.J. Lupien et al., Lancet 1978, 1, 283).

In GB-A-2 162 179 werden Naphthyl-Analoga der Mevalonsäure bzw. von Mevalonolakton, welche die HMG-CoA-Reduktase hemmen, beschrieben. Die U.S. Patente 4,459,422 und 4,567,289 betreffen substituierte 4-Hydroxytetrahydropyran-2-one, die aufgrund ihrer hemmenden Wirkung auf die HMG-CoA-Reduktase die Cholesterinsynthese inhibieren. Verbindungstypen, wie durch die nachstehenden Formeln I und II charakterisiert, werden in keiner der genannten Schriften aufgezeigt. Den Verbindungen des Standes der Technik fehlt insbesondere das Brückenglied A-B.

Es wurde nun gefunden, daß Dihydroxycarbonsäuren der allgemeinen Formel I sowie die entsprechenden Laktone der Formel II Hemmstoffe der HMG-CoA-Reduktase sind.

Die Erfindung betrifft daher 3,5-Dihydroxycarbonsäuren und deren Derivate der allgemeinen Formel I

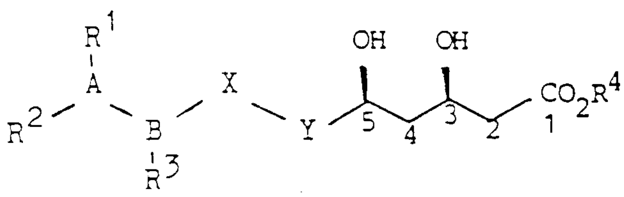

I

und die entsprechenden Laktone der Formel II

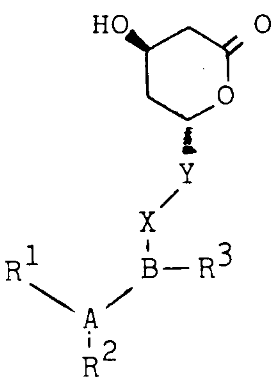

II

In den allgemeinen Formeln I und II bedeuten

A-B CH-CH oder C=C,
X-Y $CH_2$-$CH_2$ oder HC=CH,

$R^1$ und $R^2$, wobei $R^1$ und $R^2$ gleich oder verschieden sind, einen gesättigten oder ungesättigten Alkylrest mit bis zu 20 C-Atomen, der mit einer Alkoxygruppe mit 1-6 C-Atomen oder der Gruppe

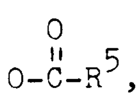

wobei $R^5$ Alkyl mit 1-8 C-Atomen bedeutet, substituiert sein kann, einen Cycloalkylrest mit 3-7 C-Atomen,

einen Phenyl, Thiophen-, Furan- oder Naphtalinrest, wobei die aromatischen Reste im Kern 1-3 fach substituiert sein können mit Halogen, Alkyl- oder Alkoxy mit jeweils 1-6 C-Atomen, Cycloalkyl mit 3-7 C-Atomen oder der Gruppe

$$O-\overset{\overset{\displaystyle O}{\|}}{C}-R^5$$

wobei $R^5$ Alkyl mit 1-8 C-Atomen bedeutet, oder einen Pyridinrest, der im Kern 1-3 fach substituiert sein kann mit Alkyl mit 1-4 C-Atomen,
$R^3$ einen gesättigten oder ungesättigten Alkylrest mit bis zu 8 C-Atomen, einen Benzylrest, welcher im Kern 1-3 fach substituiert sein kann mit Halogen, Alkoxy oder Alkyl mit jeweils 1-4 C-Atomen, einen Phenyl-, Thiophen-, Furan-, Naphtalinrest, wobei die aromatischen Reste im Kern 1-3 fach substituiert sein können mit Halogen, Alkoxy oder Alkyl mit jeweils 1-4 C-Atomen, oder einen Cycloalkylrest mit 3-7 C-Atomen
$R^4$ in Formel I Wasserstoff, Alkyl mit 1-8 C-Atomen, ein Metallkation, ein Ammoniumion, Phenyl, Benzyl oder den 2,3-Dihydroxypropylrest.

Bevorzugt bedeuten in den allgemeinen Formeln I und II

A-B CH-CH oder C = C
X-Y $CH_2$-$CH_2$ oder HC = CH

$R^1$ und $R^2$, wobei $R^1$ und $R^2$ gleich oder verschieden sind, einen Alkylrest mit 1-10 C-Atomen, der mit einer Alkoxygruppen mit 1-6 C-Atomen oder der Gruppe

$$O-\overset{\overset{\displaystyle O}{\|}}{C}-R^5,$$

wobei $R^5$ Alkyl mit 1-8 C-Atomen bedeutet, substituiert sein kann, einen Cycloalkylrest mit 5-6 C-Atomen, einen Phenylrest welcher im Kern 1-3 fach substituiert sein kann mit Halogen, Alkyl oder Alkoxy mit jeweils 1-4 C-Atomen, Cycloalkyl mit 5-6 C-Atomen, oder der Gruppe

$$O-\overset{\overset{\displaystyle O}{\|}}{C}-R^5,$$

wobei
$R^5$ Alkyl mit 1-8 C-Atomen bedeutet,
$R^3$ einen Alkylrest mit 1-8 C-Atomen, einen Benzylrest, welcher im Kern 1-3 fach substituiert sein kann mit Halogen, Alkoxy oder Alkyl mit jeweils 1-4 C-Atomen, einen Phenylrest welcher im Kern 1-3 fach substituiert sein kann mit Halogen, Alkoxy oder Alkyl mit jeweils 1-4 C-Atomen, oder einen Cycloalkylrest mit 5-6 C-Atomen,
$R^4$ in Formel I Wasserstoff, Alkyl mit 1-6 C-Atomen, ein Metallkation, ein Ammoniumion, Phenyl oder Benzyl.

Besonders bevorzugt bedeuten in den allgemeinen Formeln I und II

A-B C = C,
X-Y HC = CH,

$R^1$ und $R^2$, wobei $R^1$ und $R^2$ gleich oder verschieden sind, einen Phenylrest, welcher im Kern 1-3 fach substituiert sein kann mit Halogen, Alkyl oder Alkoxy mit jeweils 1 bis 4 C-Atomen oder der Gruppe

$$-O-\overset{\overset{\displaystyle O}{\|}}{C}-R^5$$

wobei $R^5$ Alkyl mit 1-8 C-Atomen bedeutet,
$R^3$ einen Alkylrest mit 1-8 C-Atomen oder einen Cycloalkylrest mit 5-6 C-Atomen,
$R^4$ in Formel I Wasserstoff, Alkyl mit 1-8 C-Atomen, ein Natrium- oder Kaliumkation oder ein Ammoniumion.

Die Verbindungen der Formel I sind bevorzugt.

Die Erfindung betrifft die reinen Enantiomeren sowie die Racemate der Formel I und Mischungen aus diesen, also die Racemate mit der absoluten Konfiguration
3R/5S bzw. 3S/5R für X-Y gleich HC = CH und
3R/5R bzw. 3S/5S für X-Y gleich $H_2C$-$CH_2$
sowie die reinen Enantiomeren
3R/5S für X-Y gleich HC = CH und
3R/5R für X-Y gleich $CH_2$-$CH_2$.

Die Erfindung betrifft ferner die reinen Enantiomeren sowie die Racemate der allgemeinen Formel II, die aus den oben genannten stereoisomeren offenkettigen Dihydroxycarbonsäuren der allgemeinen Formel I hervorgehen. Im einzelnen sind das die Racemate mit den absoluten Konfigurationen
3R/5S bzw. 3S/5R für X-Y gleich HC = CH und
3R/5R bzw. 3S/5S für X-Y gleich $CH_2$-$CH_2$
sowie die reinen Enantiomeren
3R/5S für X-Y gleich HC = CH und
3R/5R für X-Y gleich $CH_2$-$CH_2$

Die Erfindung betrifft ferner ein Verfahren zur Herstellung der Verbindungen der allgemeinen Formeln I und II, das dadurch gekennzeichnet ist, daß man

a) entsprechend substituierte Aldehyde der Formel III

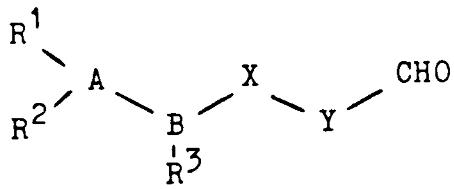

III

worin A-B, X-Y, $R^1$,$R^2$ und $R^3$ die angegebenen Bedeutungen haben, in die entsprechenden Hydroxyketoester der allgemeinen Formel IV überführt

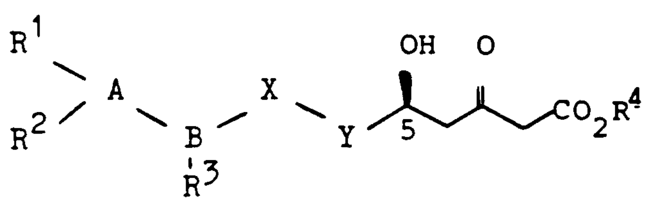

IV

worin A-B, X-Y, $R^1$,$R^2$ und $R^3$ die angegebenen Bedeutungen haben und $R^4$ Alkyl mit 1-8 C-Atomen bedeutet,

b) die Hydroxyketoester der Formel IV in die entsprechenden 3,5-Dihydroxyverbindungen der Formel I

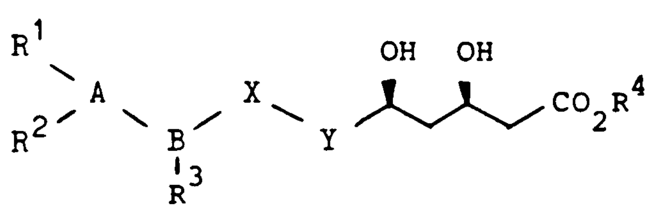

I

worin A-B, X-Y, $R^1$,$R^2$ und $R^3$ die zu Formel I angegebenen Bedeutungen haben, und $R^4$ Alkyl mit 1-8 C-Atomen ist, überführt und eine erhaltene Verbindung gegebenenfalls verseift zu einer Verbindung der Formel I, worin $R^4$ ein Metallkation darstellt, daraus gegebenenfalls die freie Säure ($R^4$ = Wasserstoff) in

Freiheit setzt und die freie Säure gegebenenfalls in Verbindungen der Formel I mit $R^4$ = Ammoniumion, Phenyl, Benzyl oder 2,3-Dihydroxypropyl überführt,
c) und eine erhaltene Verbindung der Formel I gegebenenfalls in ein Lakton der Formel II überführt,

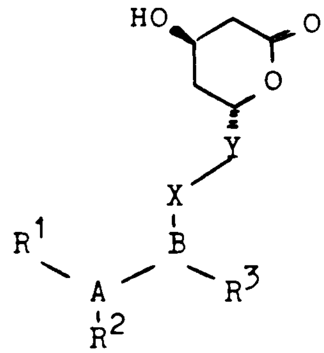

II

worin A-B, X-Y, $R^1$,$R^2$ und $R^3$ die angegebenen Bedeutungen haben.

Die Umwandlung von Verbindungen der Formel III in Verbindungen der Formel IV erfolgt je nach Gegebenheiten und Erfordernissen nach verschiedenen Varianten, wie z.B.

1. Reaktion des Dianions von Acetessigestern mit Aldehyden der Formel III führt in Lösungsmitteln wie THF bei -78 °C bis Raumtemperatur zu racemischen Verbindungen der Formel IV. Dianionen von Acetessigestern können mit verschiedenen Basen, vorzugsweise Natriumhydrid und Lithiumdiisopropylamid (LDA) vorzugsweise in THF bei -40 ° bis Raumtemperatur bereitet werden.

2. Umsetzung der Enolate von achiralen Essigsäureestern, wie z.B. Ethyl oder Propylestern, die mit starken Basen, vorzugsweise LDA, in THF bereitet werden, mit Aldehyden der Formel III führt in Lösungsmitteln wie beispielsweise THF bei Temperaturen zwischen -78 °C und 0 °C zu racemischen Verbindungen der Formel V,

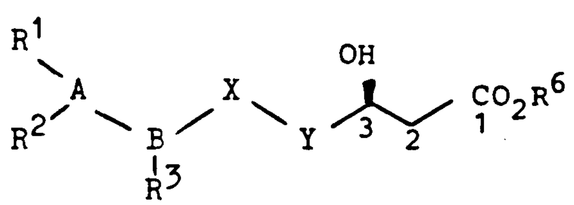

V

worin $R^6$ eine achirale Säureschutzgruppe, wie z.B. die Ethyl- oder Propylgruppe, bedeutet. Umsetzung mit einem weiteren Essigsäureesterenolat führt in Lösungsmitteln wie beispielsweise THF bei -78 °C bis Raumtemperatur zu racemischen Verbindungen der Formel IV.

3. Umsetzung von Aldehyden der Formel III mit Lithium-, Natrium-, Kalium- oder Magnesiumenolaten von optisch aktiven Essigsäureestern führt in Lösungsmitteln wie THF bei -78 °C bis 0 °C zu optisch aktiven Addukten der Formel V. In diesem Fall bedeutet $R^6$ eine geeignete optisch aktive Säureschutzgruppe, die die Stereochemie an 3-C bestimmt. Vorzugsweise wird hier die Gruppe

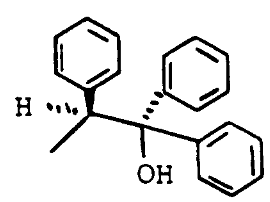

verwendet, die nach M. Braun und R. Devant (Tetrahedron Lett. 25, 5031 (1984)) die 3 R Konfiguration ergibt und aus L-(+)-Mandelsäure hergestellt wird. Es eignen sich aber auch andere chirale optisch aktive Gruppen. Die nun optisch aktiven Verbindungen der Formel V werden mit achiralen Essigsäureesterenolaten nach Variante 2 entweder direkt in die jetzt optisch aktiven Verbindungen der Formel IV überführt oder nach Überführung der primären Addukte in die entsprechenden Alkylester, vorzugsweise Methylester.

Die Umwandlung von Verbindungen der Formel IV in Verbindungen der Formel I erfolgt z.B. in Analogie zu einem literaturbekannten Verfahren (K. Narasaka und H.C. Pai, Chemistry Lett. 1980 1415). Zunächst wird mit einem Trialkylboran, vorzugsweise Triethylboran, in THF bei Raumtemperatur umgesetzt und dann bei -78 °C bis 0 °C mit Natriumborhydrid, gegebenenfalls unter Zusatz von Methanol reduziert. Auf diese Weise erhält man die in den vorhergehenden Ausführungen beschriebenen stereochemischen Verhältnisse.

Die racemischen Verbindungen der Formeln I und II können nach den bekannten Verfahren der Racematspaltung in die reinen Enantiomeren getrennt werden. Die Salze und Säuren der Verbindungen der allgemeinen Formel I werden nach den allgemein bekannten Methoden erhalten.

Das beschriebene Verfahren beinhaltet die Herstellung von Endprodukten und Zwischenprodukten mit A-B gleich CH-CH und C=C sowie X-Y gleich $CH_2$-$CH_2$ und HC=CH. Die Hydrierung gegebenenfalls vorhandener Doppelbindungen kann gegebenenfalls entweder an Zwischenprodukten oder an den Endprodukten nach üblichen Verfahren vorgenommen werden.

Verbindungen der Formel I, für die gilt A-B gleich CH-CH und X-Y gleich $CH_2$-$CH_2$, werden z.B. durch katalytische Hydrierung aus Verbindungen der Formel I gewonnen, worin A-B gleich CH-CH und X-Y gleich HC=CH sind. Die Hydrierung wird in Lösungsmitteln wie Methanol, Ethanol oder Essigester mit Katalysatoren wie Pd/C bei Normaldruck oder erhöhtem Druck ausgeführt.

Die Laktone der Formel II werden ebenfalls nach bekannten Verfahren erhalten beispielsweise durch Wasserabspaltung aus den offenen Dihydroxycarbonsäuren der Formel I, $R^4$ gleich H, in Benzol, Hexan oder Toluol unter Zusatz von p-Toluolsulfonsäure bei Raumtemperatur bis Rückflußtemperatur.

Die Aldehyde der Formel III werden z.B. aus den Nitrilen der Formel VI,

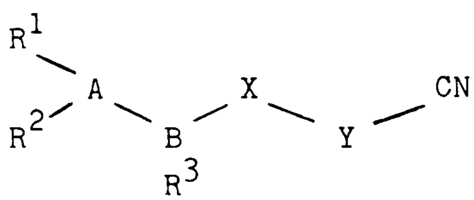

VI

gewonnen. Die Reaktion der Nitrile der Formel VI, worin A-B, X-Y, $R^1$,$R^2$ und $R^3$ die zu Formel I angegebene Bedeutung haben, mit Diisobutylaluminiumhydrid (DIBAH) in THF bei -10 °C bis 50 °C führt nach Hydrolyse direkt zu den Aldehyden der Formel III.

Die Nitrile der formel VI werden aus den Aldehyden der Formel VII gewonnen,

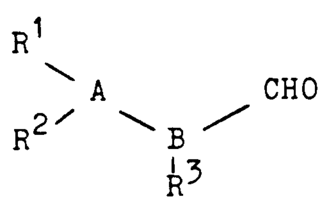

VII

worin A-B, $R^1$ und $R^3$ die angegebene Bedeutung haben, und zwar durch Umsetzung mit Cyanomethanphosphonaten, insbesondere Cyanomethanphosphonsäurediisopropylester in Lösungsmitteln wie THF in Gegenwart einer Base, vorzugsweise Natriumhydrid bei Temperaturen zwischen -20 °C und Raumtemperatur. Es fallen überwiegend die E-Isomeren an.

Die Aldehyde der Formel VII werden durch Oxidation der Alkohole der Formel VIII gewonnen,

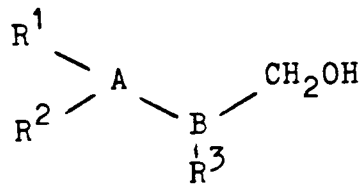

VIII

worin A-B, $R^1$,$R^2$ und $R^3$ die angegebene Bedeutung haben. Vorzugsweise wird die Oxidation mit Pyridiniumchlorochromat (PCC) in Dichlormethan bei Raumtemperatur ausgeführt.

Die Alkohole der Formel VIII entstehen durch Reduktion aus den Estern der Formel IX,

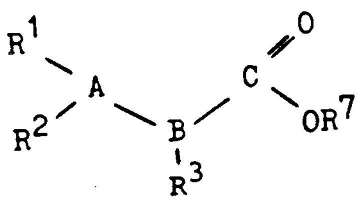

IX

worin A-B, $R^1$,$R^2$ und $R^3$ die angegebenen Bedeutungen haben. $R^7$ bedeutet eine geeignete Säureschutzgruppe, vorzugsweise Alkyl mit 1-5 C-Atomen. Die Reduktion zu Verbindungen der Formel VIII wird vorzugsweise mit DIBAH in Dichlormethan oder Lithiumaluminiumhydrid in Ether oder THF bei -40 °C bis Raumtemperatur ausgeführt.

Zur Darstellung von Verbindungen der Formel IX geht man aus von Ketonen der Formel X

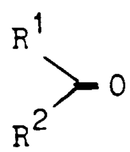

X

worin $R^1$ und $R^2$ die angegebene Bedeutung haben. Diese werden mit geeigneten Enolaten von Carbonsäureestern der Formel XI,

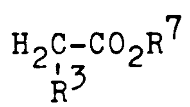

XI

worin $R^3$ die angegebene Bedeutung hat und $R^7$ eine Schutzgruppe, insbesondere Alkyl mit 1-5 C-Atomen, bedeutet, umgesetzt. Vorzugsweise werden die Enolate mit LDA in THF bei -78 °C, bereitet. Die Reaktion ergibt Additionsprodukte der Formel XII

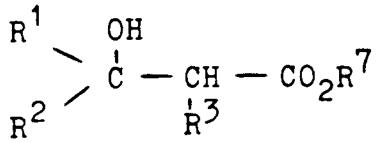

XII

worin $R^1$, $R^2$, $R^3$ und $R^7$ die angegebene Bedeutung haben. Elimination von Wasser vorzugsweise mit p-Toluolsulfonsäure in Toluol unter erhöhter bis Rückflußtemperatur führt in den ungesättigten Estern der Formel IX mit A-B gleich C = C.

Bei der Umwandlung von XII in IX, A-B gleich C-C, entstehen falls $R^1$ ungleich $R^2$ Diastereomere. Diese

werden auf der Stufe IX z.B. durch Flash-Chromatographie oder HPLC getrennt.

In bestimmten Fällen erfolgt die Trennung erst auf einer späteren Stufe oder auf der Endstufe.

Die gesättigten Verbindungen der Formel IX, A-B gleich CH-CH, werden z.B. durch katalytische Hydrierung aus den ungesättigten A-B gleich C = C erhalten, vorzugsweise mit Pd/C in Lösungsmitteln wie Methanol, Ethanol oder Eisessig bei erhöhtem Druck.

Die als Ausgangsmaterial verwendeten Carbonsäureester der Formel XI sind gut zugänglich. Die verwendeten Ketone X sind nur teilweise auf dem Markt. Ein allgemeines Verfahrens zu ihrer Herstellung besteht in der Umsetzung eines Grignard-Reagenzes $R^1$-MgHal mit einem Aldehyd $R^2$-CHO (Houben-Weyl, Methoden, der Organischen Chemie, Band 6/1a/2, S. 928, 1980) wobei $R^1$ und $R^2$ die angegebene Bedeutung haben, zu Verbindungen der Formel XIII,

$$R^1 - \overset{OH}{\overset{|}{C}}H - R^2 \qquad XIII$$

Die Alkohole der Formel XIII werden dann zu den Ketonen der Formel X oxidiert, vorzugsweise mit Pyridiniumchlorochromat (PCC) in Dichlormethan bei Raumtemperatur. Ein weiteres Verfahren zur Herstellung von Verbindungen der Formel X ist die Friedel-Crafts-Acylierung (Houben-Weyl, Methoden der Organischen Chemie Band 7/2a, Seite 15, 1973)

Die Reinigung von Zwischenprodukten erfolgt falls notwendig durch Destillation, Kristallisation, Flash-Chromatographie oder HPLC.

Außer den in den Beispielen beschriebenen Verbindungen lassen sich nach dem erfindungsgemäßen Verfahren die folgenden Verbindungen herstellen.

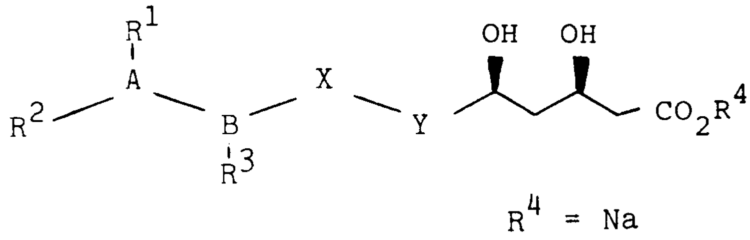

| $R^1$ | $R^2$ | $R^3$ | A-B | X-Y |
|---|---|---|---|---|
| 4-Fluorphenyl | 4-Fluorphenyl | Methyl | C=C | HC=CH |
| " | " | Ethyl | " | " |
| " | " | t-Butyl | " | " |
| 4-Fluor-3-methylphenyl | 4-Fluor-3-methylphenyl | Methyl | " | " |
| " | " | Ethyl | " | " |
| " | " | Isobutyl | " | " |
| " | " | t-Butyl | " | " |
| 4-Fluor-2-methylphenyl | 4-Fluor-2-methylphenyl | Methyl | " | " |
| " | " | Ethyl | " | " |
| " | " | Isobutyl | " | " |
| " | " | t-Butyl | " | " |
| 4-Fluor-3-methylphenyl | 4-Methoxyphenyl | Methyl | " | " |
| " | " | Ethyl | " | " |
| " | " | Isopropyl | " | " |
| " | " | Isobutyl | " | " |
| " | " | t-Butyl | " | " |
| 4-Methoxyphenyl | 4-Fluor-3-methylphenyl | Methyl | " | " |
| " | " | Ethyl | " | " |
| " | " | Isopropyl | " | " |
| " | " | Isobutyl | " | " |
| " | " | t-Butyl | " | " |
| 4-Fluor-2-methylphenyl | 4-Methoxyphenyl | Methyl | " | " |
| " | " | Ethyl | " | " |
| " | " | Isopropyl | " | " |
| " | " | Isobutyl | " | " |
| " | " | t-Butyl | " | " |

| $R^1$ | $R^2$ | $R^3$ | A-B | X-Y |
|---|---|---|---|---|
| 4-Methoxyphenyl | 4-Fluor-2-methylphenyl | Methyl | C=C | HC=CH |
| " | " | Ethyl | " | " |
| " | " | Isopropyl | " | " |
| " | " | Isobutyl | " | " |
| " | " | t-Butyl | " | " |
| 3,4,5-Trimethoxyphenyl | 4-Fluorphenyl | Methyl | " | " |
| " | " | Ethyl | " | " |
| " | " | Isopropyl | " | " |
| " | " | Isobutyl | " | " |
| 4-Fluorphenyl | 3,4,5-Trimethoxyphenyl | Methyl | " | " |
| " | " | Ethyl | " | " |
| " | " | Isopropyl | " | " |
| " | " | Isobutyl | " | " |
| 4-Fluor-2-methylphenyl | 3,4,5-Trimethoxyphenyl | Methyl | " | " |
| " | " | Ethyl | " | " |
| " | " | Isopropyl | " | " |
| " | " | Isobutyl | " | " |
| 3,4,5-Trimethoxyphenyl | 4-Fluor-2-methylphenyl | Methyl | " | " |
| " | " | Ethyl | " | " |
| " | " | Isopropyl | " | " |
| " | " | Isobutyl | " | " |
| 3,4,5-Trimethoxyphenyl | 4-Fluor-3-methylphenyl | Methyl | " | " |
| " | " | Ethyl | " | " |
| " | " | Isopropyl | " | " |
| " | " | Iosbutyl | " | " |
| 4-Fluor-3-methylphenyl | 3,4,5-Trimethoxyphenyl | Methyl | " | " |
| " | " | Ethyl | " | " |
| " | " | Isopropyl | " | " |
| " | " | Isobutyl | " | " |

| $R^1$ | $R^2$ | $R^3$ | A-B | X-Y |
|---|---|---|---|---|
| 4-Fluorphenyl | 4-Fluorphenyl | Methyl | HC-CH | HC=CH |
| " | " | Ethyl | " | " |
| " | " | t-Butyl | " | " |
| 4-Fluor-3-methylphenyl | 4-Fluor-3-methylphenyl | Methyl | " | " |
| " | " | Ethyl | " | " |
| " | " | Isobutyl | " | " |
| " | " | t-Butyl | " | " |
| 4-Fluor-2-methylphenyl | 4-Fluor-2-methylphenyl | Methyl | " | " |
| " | " | Ethyl | " | " |
| " | " | Isobutyl | " | " |
| " | " | t-Butyl | " | " |
| 4-Fluor-3-methylphenyl | 4-Methoxyphenyl | Methyl | " | " |
| " | " | Ethyl | " | " |
| " | " | Isopropyl | " | " |
| " | " | Isobutyl | " | " |
| " | " | t-Butyl | " | " |
| 4-Methoxyphenyl | 4-Fluor-3-methylphenyl | Methyl | " | " |
| " | " | Ethyl | " | " |
| " | " | Isopropyl | " | " |
| " | " | Isobutyl | " | " |
| " | " | t-Butyl | " | " |
| 4-Fluor-2-methylphenyl | 4-Methoxyphenyl | Methyl | " | " |
| " | " | Ethyl | " | " |
| " | " | Isopropyl | " | " |
| " | " | Isobutyl | " | " |
| " | " | t-Butyl | " | " |
| 4-Methoxyphenyl | 4-Fluor-2-methylphenyl | Methyl | " | " |
| " | " | Ethyl | " | " |
| " | " | Isopropyl | " | " |
| " | " | Isobutyl | " | " |
| " | " | t-Butyl | " | " |

| $R^1$ | $R^2$ | $R^3$ | A-B | X-Y |
|---|---|---|---|---|
| 3,4,5-Trimethoxyphenyl | 4-Fluorphenyl | Methyl | HC-CH | HC=CH |
| " | " | Ethyl | " | " |
| " | " | Isopropyl | " | " |
| " | " | Isobutyl | " | " |
| 4-Fluorphenyl | 3,4,5-Trimethoxyphenyl | Methyl | " | " |
| " | " | Ethyl | " | " |
| " | " | Isopropyl | " | " |
| " | " | Isobutyl | " | " |
| 4-Fluor-2-methylphenyl | 3,4,5-Trimethoxyphenyl | Methyl | " | " |
| " | " | Ethyl | " | " |
| " | " | Isopropyl | " | " |
| " | " | Isobutyl | " | " |
| 3,4,5-Trimethoxyphenyl | 4-Fluor-2-methylphenyl | Methyl | " | " |
| " | " | Ethyl | " | " |
| " | " | Isopropyl | " | " |
| " | " | Isobutyl | " | " |
| 3,4,5-Trimethoxyphenyl | 4-Fluor-3-methylphenyl | Methyl | " | " |
| " | " | Ethyl | " | " |
| " | " | Isopropyl | " | " |
| " | " | Iosbutyl | " | " |
| 4-Fluor-3-methylphenyl | 3,4,5-Trimethoxyphenyl | Methyl | " | " |
| " | " | Ethyl | " | " |
| " | " | Isopropyl | " | " |
| " | " | Isobutyl | " | " |

| $R^1$ | $R^2$ | $R^3$ | A-B | X-Y |
|---|---|---|---|---|
| 4-Fluorphenyl | 4-Fluorphenyl | Methyl | HC-CH | $H_2C-CH_2$ |
| " | " | Ethyl | " | " |
| " | " | t-Butyl | " | " |
| 4-Fluor-3-methylphenyl | 4-Fluor-3-methylphenyl | Methyl | " | " |
| " | " | Ethyl | " | " |
| " | " | Isobutyl | " | " |
| " | " | t-Butyl | " | " |
| 4-Fluor-2-methylphenyl | 4-Fluor-2-methylphenyl | Methyl | " | " |
| " | " | Ethyl | " | " |
| " | " | Isobutyl | " | " |
| " | " | t-Butyl | " | " |
| 4-Fluor-3-methylphenyl | 4-Methoxyphenyl | Methyl | " | " |
| " | " | Ethyl | " | " |
| " | " | Isopropyl | " | " |
| " | " | Isobutyl | " | " |
| " | " | t-Butyl | " | " |
| 4-Methoxyphenyl | 4-Fluor-3-methylphenyl | Methyl | " | " |
| " | " | Ethyl | " | " |
| " | " | Isopropyl | " | " |
| " | " | Isobutyl | " | " |
| " | " | t-Butyl | " | " |
| 4-Fluor-2-methylphenyl | 4-Methoxyphenyl | Methyl | " | " |
| " | " | Ethyl | " | " |
| " | " | Isopropyl | " | " |
| " | " | Isobutyl | " | " |
| " | " | t-Butyl | " | " |
| 4-Methoxyphenyl | 4-Fluor-2-methylphenyl | Methyl | " | " |
| " | " | Ethyl | " | " |
| " | " | Isopropyl | " | " |
| " | " | Isobutyl | " | " |
| " | " | t-Butyl | " | " |

| $R^1$ | $R^2$ | $R^3$ | A-B | X-Y |
|---|---|---|---|---|
| 3,4,5-Trimethoxyphenyl | 4-Fluorphenyl | Methyl | HC-CH | $H_2C-CH_2$ |
| " | " | Ethyl | " | " |
| " | " | Isopropyl | " | " |
| " | " | Isobutyl | " | " |
| 4-Fluorphenyl | 3,4,5-Trimethoxyphenyl | Methyl | " | " |
| " | " | Ethyl | " | " |
| " | " | Isopropyl | " | " |
| " | " | Isobutyl | " | " |
| 4-Fluor-2-methylphenyl | 3,4,5-Trimethoxyphenyl | Methyl | " | " |
| " | " | Ethyl | " | " |
| " | " | Isopropyl | " | " |
| " | " | Isobutyl | " | " |
| 3,4,5-Trimethoxyphenyl | 4-Fluor-2-methylphenyl | Methyl | " | " |
| " | " | Ethyl | " | " |
| " | " | Isopropyl | " | " |
| " | " | Isobutyl | " | " |
| 3,4,5-Trimethoxyphenyl | 4-Fluor-3-methylphenyl | Methyl | " | " |
| " | " | Ethyl | " | " |
| " | " | Isopropyl | " | " |
| " | " | Iosbutyl | " | " |
| 4-Fluor-3-methylphenyl | 3,4,5-Trimethoxyphenyl | Methyl | " | " |
| " | " | Ethyl | " | " |
| " | " | Isopropyl | " | " |
| " | " | Isobutyl | " | " |

Das Enzym HMG-CoA-Reduktase ist in der Natur weit verbreitet. Es katalysiert die Bildung von Mevalonsäure aus HMG-CoA. Diese Reaktion ist ein zentraler Schritt der Cholesterin-Biosynthese (I.R. Sabine, 3-Hydroxy-3-methylgluaryl Coenzym A Reductase, CRC Press, 1983). Hohe Cholesterinspiegel werden mit einer Reihe von Erkrankungen wie z.B. koronarer Herzkrankheit oder Atherosklerose in Verbindung gebracht. Daher ist die Senkung erhöhter Cholesterinspiegel zur Vorbeugung und Behandlung solcher Erkrankungen ein therapeutisches Ziel. Ein Ansatzpunkt liegt in der Hemmung bzw. Verminderung der endogenen Cholesterinsynthese. Hemmstoffe der HMG-CoA-Reduktase blockieren die Cholesterin-Biosynthese aus einer frühen Stufe. Sie eignen sich daher zur Vorbeugung und Behandlung von Erkrankungen, die durch einen erhöhten Cholesterinspiegel verursacht werden. Eine Reduktion bzw. Verminderung der endogenen Synthese führt zu einer erhöhten Aufnahme von Cholesterin aus dem Plasma in die Zellen. Ein zusätzlicher Effekt läßt sich durch gleichzeitige Gabe Gallensäuren-bindender Stoffe wie Anionenaustauscher erzielen. Die erhöhte Gallensäurenausscheidung führt zu einer verstärkten Neusynthese und damit zu einem erhöhten Cholesterinabbau (M.S. Brown, P.T. Kovanen, J.L. Goldstein, Science 212, 628 (1981); M.S. Brown, J.L. Goldstein, Spektrum der Wissenschaften 1985 (1), 96). Die erfindungsgemäßen Verbindungen sind Hemmstoffe der HMG-CoA-Reduktase. Sie eignen sich daher zur Hemmung bzw. Verminderung der Cholesterin-Biosynthese und damit zur Vorbeugung oder Behandlung von Erkrankungen, die durch erhöhte Cholesterinspiegel verursacht werden, insbesondere koronare Herzkrankheit, Atherosklerose, Hypercholesterinämie, Hyperlipoproteinämie und ähnliche Erkrankungen.

Die Erfindung betrifft daher auch pharmazeutische Präparate auf Basis der Verbindungen der Formel I bzw. der Formel II sowie die Verwendung dieser Verbindungen als Arzneimittel, insbesondere zur Behandlung der Hypercholesterinämie.

Die Verbindungen der Formeln I und II werden in verschiedenen Dosierungsformen verabreicht, vorzugsweise oral in Form von Tabletten, Kapseln oder Flüssigkeiten. Die tägliche Dosis bewegt sich je nach Körpergewicht und Konstitution des Patienten im Bereich von 3 mg bis 2500 mg, vorzugsweise jedoch im Dosisbereich 10 - 500 mg. Die erfindungsgemäßen Verbindungen können zur Anwendung kommen gelöst oder suspensidert in pharmakologisch unbedenklichen organischen Lösungsmitteln, wie ein- oder mehrwertigen Alkoholen, wie z.B. Ethanol oder Glycerin, in Triacetin, Ölen wie z.B. Sonnenblumenöl der Lebertran, Ethern, wie z.B. Diethylenglykoldimethylether oder auch Polyethern, wie z.B. Polyethylenglykol, oder auch in Gegenwart anderer pharmakologisch unbedenklicher Polymerträger, wie z.B. Polyvinylpyrrolidon, oder anderen pharmazeutisch annehmbaren Zusatzstoffen wie Stärke, Cyclodextrin oder Polysacchariden. Ferner können die erfindungsgemäßen Verbindungen kombiniert werden mit Zusatzstoffen, die Gallensäuren binden, insbesondere nicht toxische, basische Anionenaustauscherharze, die Gallensäure in einer nichtresorbierbaren Form im Gastrointestinaltrakt binden. Die Salze der Dihydroxycarbonsäuren können auch als wäßrige Lösungen verabreicht werden.

Die Verbindungen der Formeln III, IV, VI, VII, VIII, IX und XII sind neu und z.B. wertvolle Zwischenprodukte für die Herstellung von Verbindungen der Formel I. Die Erfindung betrifft daher auch die Verbindung der Formeln III, IV, VI, VII, VIII, IX und XII sowie Verfahren zu deren Herstellung.

Die HMG-CoA-Reduktase-Aktivität wurde in folgenden Testsystemen bestimmt:

1) Inhibierung der HMG-CoA-Reduktase-Aktivität an solubilisierten Enzympräparaten aus Ratten-Lebermikrosomen

Die HMG-CoA-Reduktase-Aktivität wurde an solubilisierten Enzympräparationen aus Lebermikrosomen von Ratten gemessen, die nach Umstellung im Tag-Nacht-Rhytmus mit Cholestyramin ($^{(R)}$Cuemid) induziert wurden. Als Substrat dient (S,R)$^{14}$C-HMG-CoA, die Konzentration von NADPH wurde während der Inkubation durch ein regenerierendes Systems aufrechterhalten. Die Abtrennung von $^{14}$C-Mevalonat von Substrat und anderen Produkten (z.B. $^{14}$C-HMG) erfolgte über Säulenelution, wobei das Elutionsprofil jeder Einzelprobe ermittelt wurde. Auf die ständige Mitführung von $^{3}$H-Mevalonat wurde verzichtet, weil es sich bei der Bestimmung um die Relativangabe der Hemmwirkung handelt. In eine Versuchsreihe wurden jeweils die enzymfreie Kontrolle, der enzymhaltige Normalansatz (= 100 %) und solche mit Präparatezusätzen zusammen behandelt. Jeder Einzelwert wurde als Mittelwert aus 3 Parallelproben gebildet. Die Signifikanz er Mittelwertsunterschiede zwischen präparatefreien und präparatehaltigen Proben wurde nach dem t-Test beurteilt. Nach der oben beschriebenen Methode wurden von den erfindungsgemäßen Verbindungen z.B. folgende Hemmwerte auf die HMG-CoA-Reduktase ermittelt ($IC_{50}$-Wert M = molare Konzentration der Verbindung, die für eine 50 %ige Hemmung erforderlich ist).

| Verbindung gemäß Beispiel | $IC_{50}$-Wert (M) |
|---|---|
| 11a | $7.0 \times 10^{-9}$ |
| 11c | $2.0 \times 10^{-8}$ |
| 11n | $1.3 \times 10^{-8}$ |

2) Prüfung auf Cholesterin-Biosynthese-Hemmung in Zellkulturen durch $^{14}$-Präkursoreinbau in Cholesterin.

**Prinzip der Methoden:**

Monolayers von HEP G2-Zellen in lipoproteinfreiem Nährmedium wurden mit entsprechenden Konzentrationen der Prüfsubstanzen 1 Stunde vorinkubiert; nach Zugabe des $^{14}$C-markierten Präkursors $^{14}$C-Natriumacetat wurde die Inkubation für 3 Stunden fortgesetzt. Danach wurde ein Teil der Zellen alkalisch verseift bei vorheriger Zugabe eines internen Standards von $^{3}$H-Cholesterin. Die Lipide der verseiften Zellen wurden mit Chloroform-Methanol extrahiert. Dieses Lipidgemisch wurde nach Zusatz von Träger-Cholesterin präparativ dünnschichtchromatographisch getrennt, die Cholesterin-Bande nach dem Sichtbarmachen mit Jod-Dämpfen isoliert und die aus dem $^{14}$C-Präkursor gebildete Menge $^{14}$C-Cholesterins szintigraphisch

bestimmt. In einem aliquoten Teil der Zellen wurde Zellprotein bestimmt, sodaß die in der Zeiteinheit/mg Zellprotein aus $^{14}C$-Präkursor gebildete Menge $^{14}C$-Cholesterins berechnet werden kann. Zum Vergleich für die Hemmwirkung eines zugesetzten Prüfpräparates diente die Lösungsmittelkontrolle, sodaß direkt die Hemmung der Cholesterin-Biosynthese bei einer bestimmten molaren Konzentration des Prüfpräparates im Medium angegeben werden kann. In aliquoten Anteilen der Zellkultur wurde die Intaktheit der Zellkultur und die fehlende Zellschädigung durch Präparate-Einwirkung morphologisch (Lichtmikroskop) beurteilt. In der folgenden Tabelle ist die relative Wirkungsstärke zu Mevinolin, das in gleicher Weise getestet wurde, angegeben.

| Verbindung gemäß Beispiel | relative Weinstärke zu Mevinolin [%] |
|---|---|
| 11a | 100 |
| 11c | 50 |
| 11n | 30 |

**Beispiel 1**

**Allgemeine Vorschrift zur Herstellung von Verbindungen der Formel XIII:**

Zu 1 Mol Magnesium-Späne in 100 ml trockenem Ether werden 0.1 Mol eines entsprechend substituierten Brombenzols gegeben. Gegebenenfalls wird die Reaktion durch Zugabe von etwas Jod, Dibromethan oder durch Erwärmen gestartet. Nach Anspringen der Reaktion wird das restliche Brombenzol in 200 ml Ether zugetropft und zwar so, daß der Ether siedet. Nach beendeter Zugabe wird noch 1h unter Rückfluß gekocht. Unter Eiskühlung werden anschließend 0.9 Mol eines entsprechend substituierten Aldehyds $R^2$-CHO in 500 ml Ether zugetropft. Man läßt auf Raumtemperatur kommen und rührt noch 1h. Anschließend wird 2h unter Rückfluß gekocht. Die Mischung wird auf gesättigte wäßrige Ammoniumchloridlösung gegossen. Man extrahiert die wäßrige Phase mit Ether (2x) und wäscht die vereinigten organischen Phasen einmal mit gesättigter wäßriger Natriumhydrogencarbonatlösung. Trocknen mit $MgSO_4$ und Abziehen des Lösungsmittels im Vakuum ergibt die Verbindungen der Formel XIII als Feststoffe oder Öle, die gegebenenfalls durch Umkristallisation oder Flash-chromatographie gereinigt werden. In den meisten Fällen ist aber eine Reinigung nicht erforderlich.

**Tabelle 1:**

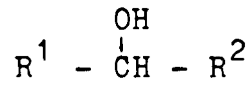

XIII

| Beispiel | Brombenzol | Aldehyd | Produkt |
|---|---|---|---|
| 1a | 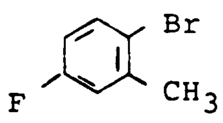 | 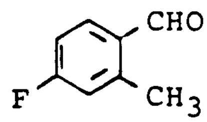 | 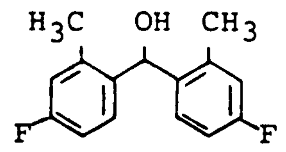 |
| 1b | 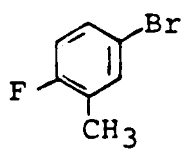 | 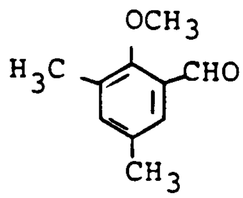 | 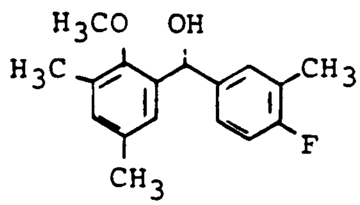 |
| 1c | 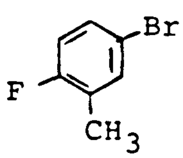 | 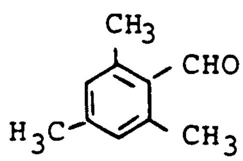 | 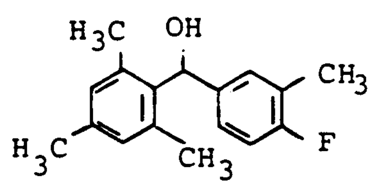 |
| 1d | 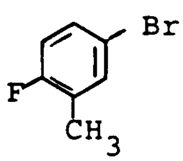 | 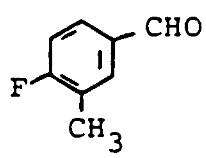 | 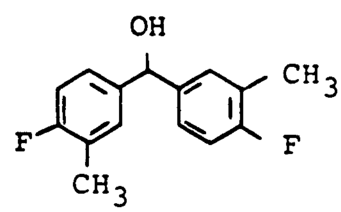 |

Beispiel 2

**Allgemeine Vorschrift zur Herstellung von Verbindungen der Formel X**

Zu einer Suspension von 0.06 Mol Pyridiniumchlorochromat (PCC) in 200 ml trockenem Methylenchlorid werden bei Raumtemperatur 0.05 Mol Alkohol der Formel XIII in 40 ml Methylenchlorid gegossen. Man läßt 3-5 h bei Raumtemperatur rühren und gibt 3 Volumen Ether zu. Filtration durch Kieselgel und Abziehen des Lösungsmittels ergibt Verbindungen der Formel X, meist in genügender Reinheit. Gegebenenfalls erfolgt eine weitere Reinigung durch Umkristallisation, Destillation oder Flash-Chromatographie.

Tabelle 2

$$R^1 - \overset{O}{\overset{\|}{C}} - R^2 \qquad X$$

| Beispiel | $R^1$ | $R^2$ | Ausgangsmaterial |
|---|---|---|---|
| 2a | 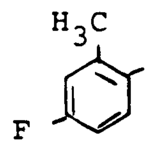 | 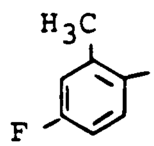 | 1a |
| 2b | 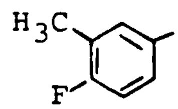 | 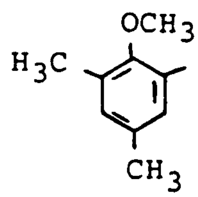 | 1b |
| 2c | 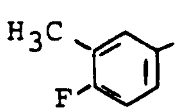 | 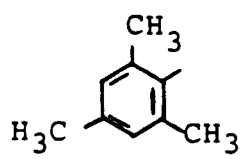 | 1c |
| 2d | 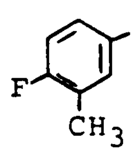 | 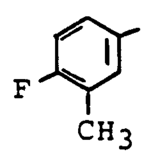 | 1d |

**Beispiel 3**
(Herstellung von Verbindungen der Formel XII)

a) 3-Hydroxy-3,3-di-(4-fluorphenyl)-2-isopropylpropionsäureethylester

50.7 ml (0.361 Mol) Diisopropylamin wurden in 250 ml trockenem Tetrahydrofuran (THF) unter Stickstoff vorgelegt. Bei 0 °C wurden 195 ml (0.314 Mol) n-BuLi (Hexan) zugetropft. 36.2 ml (0.241 Mol) Isovaleriansäureethylester zugetropft. Nach 1 h bei -70 °C wurden bei dieser Temperatur 50 g (0.229 Mol) 4,4-Difluorbenzophenon in 200 ml THF zugetropft Nach 1.5h wurde auf 1n wäßrige Salzsäure gegossen. Man extrahierte mit Ether (3x) und wusch die vereinigten organischen Phasen einmal mit gesättigter wäßriger Natriumhydrogencarbonatlösung. Trocknen mit Magnesiumsulfat und Abziehen des Lösungsmittels im Vakuum ergab 74.9 g (0.215 Mol, 94 %) Beispiel 3a, das ohne weitere Reinigung weiter umgesetzt wurde.
MS (DCI), $C_{20}H_{22}F_2O_2$ : 333 $(M+H^+)$, 331
H-NMR (60 MHz, $CDCl_3$): $\delta$ = 7.60 - 6.60(m,8H), 4.05(q,2H), 3.39(d,1H), 1.90 (m,1H), 1.20-0.80 (m, 9H)

In Analogie zu Beispiel 3a wurden die in Tabelle 3 aufgeführten Verbindungen erhalten.

Tabelle 3:

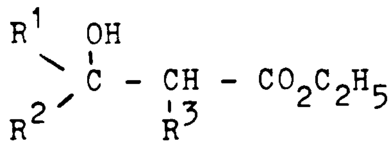

| Beispiel | $R^1$ | $R^2$ | $R^3$ | Molmasse |
|---|---|---|---|---|
| 3b | 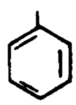 | 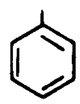 | $-CH_2CH(CH_3)_2$ | $C_{21}H_{26}O_3$, 326 |
| 3c | 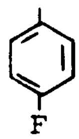 | 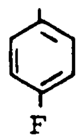 | $-CH_2CH(CH_3)_2$ | $C_{21}H_{24}F_2O_3$, 362.4 |
| 3d | 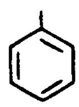 | 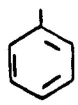 | 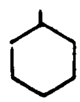 | $C_{23}H_{28}O_3$, 352 |
| 3e | 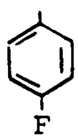 | 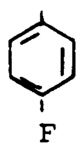 | $-(CH_2)_3CH_3$ | $C_{21}H_{24}F_2O_3$, 362,4 |
| 3f | 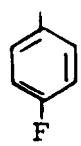 | 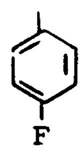 | 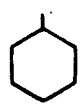 | $C_{23}H_{26}F_2O_3$, 388,4 |
| 3g | 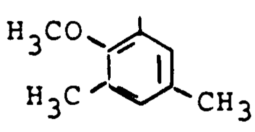 | 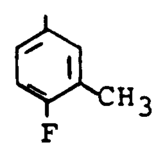 | $-CH(CH_3)_2$ | $C_{24}H_{31}FO_4$, 402,5 |
| 3h | 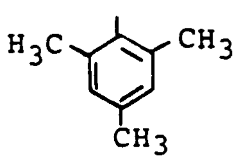 | 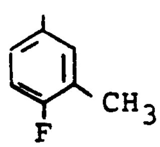 | $-CH(CH_3)_2$ | $C_{24}H_{31}FO_3$, 386,5 |

Fortsetzung Tabelle 3:

| Beispiel | $R^1$ | $R^2$ | $R^3$ | Molmasse |
|---|---|---|---|---|
| 3i | 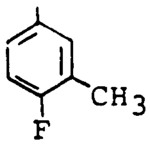 | 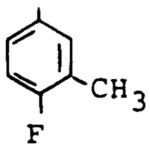 | $-CH(CH_3)_2$ | $C_{22}H_{26}F_2O_3$, 376.4 |
| 3j | 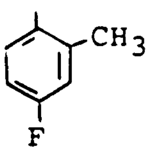 | 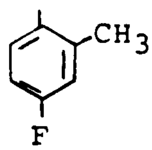 | $-CH(CH_3)_2$ | $C_{22}H_{26}F_2O_3$, 376.4 |

**Beispiel 4**

(Herstellung von Verbindungen der Formel IX mit A-B gleich C=C)

a) 3,3-Di-(4-fluorphenyl)-2-isopropylacrylsäureethylester

74.8 g (0.215 Mol) der Verbindung gemäß Beispiel 3a wurden in 800 ml Toluol gelöst, mit 7.5 g (0.04 Mol) p-Toluolsulfonsäure versetzt und 2H am Wasserabscheider unter Rückfluß erhitzt. Nach dem Abkühlen wurde die Lösung zweimal mit gesättigter wäßriger Natriumhydrogencarbonatlösung gewaschen und getrocknet ($MgSO_4$). Abziehen des Lösungsmittels im Vakuum ergab 70.8 g (0.214 Mol, 99 %) Beispiel 4a, das ohne weitere Reinigung für die nächste Stufe eingesetzt wurde.

MS, $C_{20}H_{20}F_2O_2$ : 330 $M^+$), 301

Tabelle 4

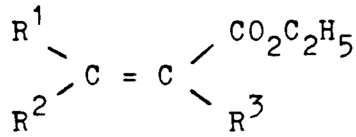

| Beispiel | $R^1$ | $R^2$ | $R^3$ | Molmasse |
|---|---|---|---|---|
| 4b | 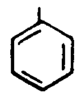 | 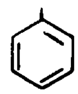 | $-CH_2CH(CH_3)_2$ | $C_{21}H_{24}O_2$<br>308(M+), 265 |
| 4c | 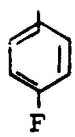 | 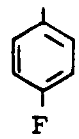 | $-CH_2CH(CH_3)_2$ | $C_{21}H_{22}F_2O_2$<br>344(M+), 301 |
| 4d | 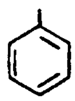 | 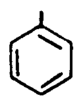 | 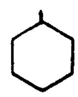 | $C_{23}H_{26}O_2$,<br>334(M+), 305 |
| 4e | 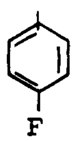 | 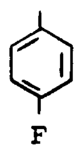 | $-(CH_2)_3CH_3$ | $C_{20}H_{20}F_2O_2$<br>330(M+), 299,<br>287 |
| 4f | 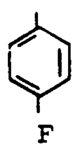 | 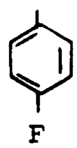 | 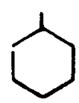 | $C_{23}H_{24}F_2O_2$<br>370(M+), 341 |
| 4g | 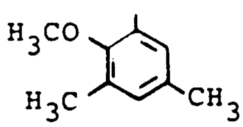 | 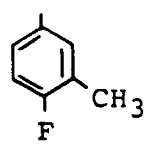 | $-CH(CH_3)_2$ | $C_{24}H_{29}FO_3$,<br>384(M+), 353 |
| 4h | 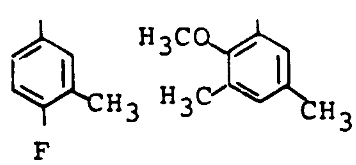 | | $-CH(CH_3)_2$ | $C_{24}H_{29}FO_3$<br>384(M+), 353 |

Fortsetzung Tabelle 4

| Beispiel | $R^1$ | $R^2$ | $R^3$ | Molmasse |
|---|---|---|---|---|
| 4i | 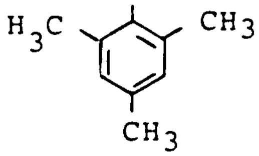 | 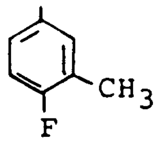 | $-CH(CH_3)_2$ | $C_{24}H_{29}FO_2$ 368($M^+$) |
| 4j | 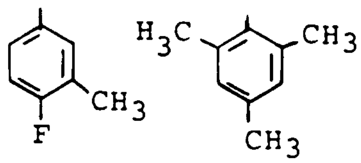 | | $-CH(CH_3)_2$ | $C_{24}H_{29}FO_2$ 368($M^+$) |
| 4k | 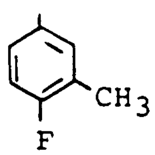 | 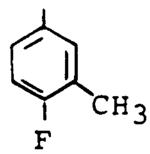 | $-CH(CH_3)_2$ | $C_{22}H_{24}F_2O_2$ 358($M^+$), 329 |
| 4l | 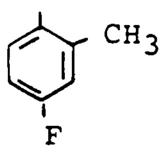 | 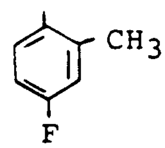 | $-CH(CH_3)_2$ | $C_{22}H_{24}F_2O_2$ 358($M^+$), 329 |

**Beispiel 4**
(Herstellung einer Verbindung der Formel IX mit A-B gleich CH-CH)

a) 3,3-Di-(4-fluorphenyl)-2-cyclohexylpropionsäureethylester

4.81 g (13 mmol) der Verbindung gemäß Beispiel 4f wurden in 120 ml Ethanol mit 500 mg Pd/C (10 %) 96h bei 150 bar hydriert. Nach beendeter Wasserstoffaufnahme wurde der Katalysator abfiltriert, das Lösungsmittel abgezogen und der Rückstand auf Kieselgel (Cyclohexan/Essigester = 19:1) chromatographiert.
Ausbeute: 4.78 g (12.8 mmol, 98 %)
Rf (Cyclohexan/Essigester = 19:1) : 0.37
MS, $C_{23}H_{26}F_2O_2$: 372 ($M^+$), 289, 203
H-NMR ($CDCl_3$, 60 MHz) :$\delta$ = 7.50-6.60(m,8H), 4.30 (d,1H), 3.90 (q,2H), 3.90 (m,1H), 3.20 (m,1H), 2.40-0.60 (m,13H)

**Beispiel 5**
(Herstellung von Verbindungen der Formel VIII)

a) 3,3-Di-(4-fluorphenyl)-2-isopropylpropenol

Zu 3.2 g (9.7 mmol) der Verbindung gemäß Beispiel 4a wurden in 100 ml Dichlormethan bei 0 °C unter Argon 24.3 ml (29,1 mmol) Diisobutylaluminiumhydridlösung (Toluol) getropft. Nach 1h bei 0 °C wurden 2ml Methanol zugetropft und anschließend 20 ml 1n wäßrige Salzsäure. Es wurde mit Ether und 1n Salzsäure bis zur Phasentrennung verdünnt. Die wäßrige Phase wurde einmal mit Ether extrahiert und die vereinigten organischen Phasen wurden mit gesättigter Natriumchloridlösung gewaschen (1x) und getrocknet ($MgSO_4$).

Abziehen des Lösungsmittels im Vakuum ergab 2.7 g (9,4 mmol, 97 %) Beispiel 5a, das ohne weitere Reinigung zur nächsten Stufe eingesetzt wurde.
Rf (Cyclohexan/Essigester = 2:1) : 0.42
MS, $C_{18}H_{18}F_2O$ : 288($M^+$), 270, 257, 245
H-NMR (60 MHz, $CDCl_3$): $\delta$ = 7.30-6.70 (m,8H), 4.05 (s,2H), 2.70 (sept.1H), 1.30 (breit, 1H), 1.03 (d,6H)

In Analogie zu Beispiel 5a wurden die Beispiele der Tabelle 5 erhalten

Tabelle 5

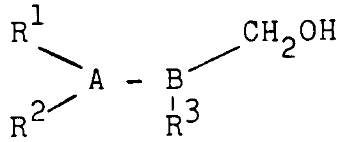

| Beispiel | $R^1$ | $R^2$ | $R^3$ | Massenspektrum |
|---|---|---|---|---|
| | | A-B gleich C=C | | |
| 5b | 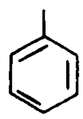 | 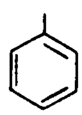 | $-CH_2CH(CH_3)_2$ | $C_{19}H_{22}O$, 266($M^+$), 209 |
| 5c | 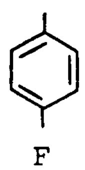 | 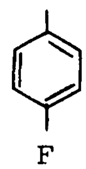 | $-CH_2CH(CH_3)_2$ | $C_{19}H_{20}F_2O$ 302($M^+$) |

Fortsetzung Tabelle 5

| Beispiel | $R^1$ | $R^2$ | $R^3$ | Massenspektrum |
|---|---|---|---|---|
| 5 d | 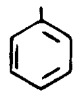 | 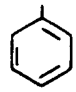 | 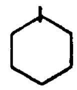 | $C_{21}H_{24}O$<br>292($M^+$)274, 261, 209 |
| 5 e | 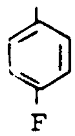 | 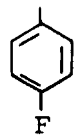 | $-(CH_2)_3CH_3$ | $C_{19}H_{20}F_2O$<br>302($M^+$), 245 |
| 5 f | 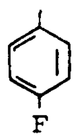 | 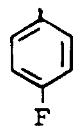 | 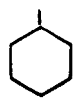 | $C_{21}H_{22}F_2O$,<br>328($M^+$),310, 297,245 |
| 5 g | 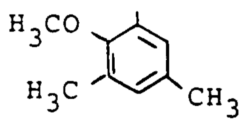 | 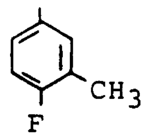 | $-CH(CH_3)_2$ | $C_{22}H_{27}FO_2$<br>342($M^+$) |
| 5 h | 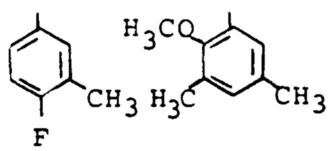 | | $-CH(CH_3)_2$ | $C_{22}H_{27}FO_2$<br>342($M^+$) |
| 5 i | 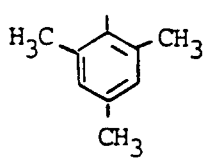 | 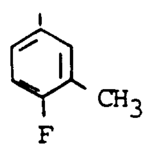 | $-CH(CH_3)_2$ | $C_{22}H_{27}FO$<br>326 ($M^+$) |
| 5 j | 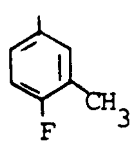 | 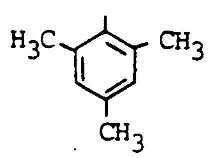 | $-CH(CH_3)_2$ | $C_{22}H_{27}FO$<br>326($M^+$) |
| 5 k | 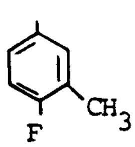 | 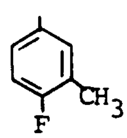 | $-CH(CH_3)_2$ | $C_{20}H_{22}F_2O$<br>316 ($M^+$) |

Fortsetzung Tabelle 5

| Beispiel | $R^1$ | $R^2$ | $R^3$ | Massenspektrum |
|---|---|---|---|---|
| 51 | 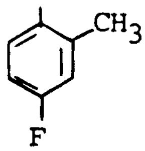 | 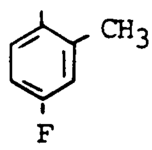 | $-CH(CH_3)_2$ | $C_{20}H_{22}F_2O$ 316($M^+$) |
| | | A-B gleich HC-CH | | |
| 5m | 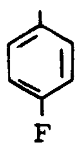 | 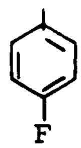 | 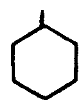 | $C_{21}H_{24}F_2O$, 330($M^+$), 312, 294, 278, 203 |

**Beispiel 6**
(Herstellung von Verbindungen der Formel VII)

a) 3,3-Di-(4-fluorphenyl)-2-isopropylpropenal

Zu einer Suspension von 3.03 g (14.1 mmol) PCC in 100 ml Dichlormethan wurden bei Raumtemperatur 2.7 g (9.4 mmol) der Verbindung gemäß Beispiel 5a in 40 ml Dichlormethan gegossen. Nach 3h Rühren bei Raumtemperatur wurde mit 3 Volumen Ether versetzt und über Kieselgel filtriert Abziehen des Lösungsmittels im Vakuum ergab 2.53 (8.8 mmol, 94 %) Beispiel 6a.
Schmp. 69 ° C
Rf (Cyclohexan/Essigester = 3:1) : 0.56
MS, $C_{18}H_{16}F_2O$: 286($M^+$), 271, 257
H-NMR (60 MHz): $\delta$ = 9.40 (d, 1H), 720-6.80 (m,8H), 2.80 (m,1H), 1.20 (d,6H).

In Analogie zu Beispiel 6a wurden die Beispiele der Tabelle 6 erhalten

**Tabelle 6**

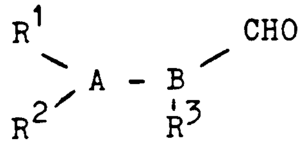

| Beispiel | $R^1$ | $R^2$ | $R^3$ | Massenspektrum |
|---|---|---|---|---|
| | | A-B gleich C=C | | |
| 6 b | 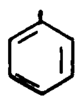 | 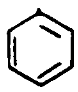 | $-CH_2CH(CH_3)_2$ | $C_{19}H_{20}O$, 264(M+) |
| 6 c | 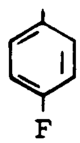 | 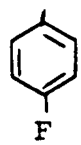 | $-CH_2CH(CH_3)_2$ | $C_{19}H_{18}F_2O$, 300(M+) |
| 6 d | 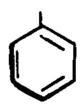 | 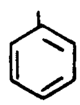 | 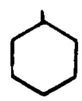 | $C_{21}H_{22}O$, 290(M+) |
| 6 e | 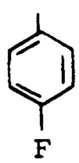 | 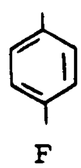 | $-(CH_2)_3CH_3$ | $C_{19}H_{18}F_2O$, 300(M+), 269 257, 244 |
| 6 f | 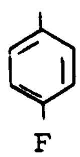 | 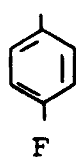 | 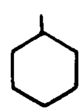 | $C_{21}H_{20}F_2O$ 326(M+), 308, 295 |
| 6 g | 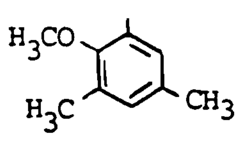 | 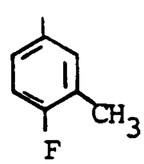 | $-CH(CH_3)_2$ | $C_{22}H_{25}FO_2$ 340(M+) |

Fortsetzung Tabelle 6

| Beispiel | $R^1$ | $R^2$ | $R^3$ | Massenspektrum |
|---|---|---|---|---|
| 6 h | 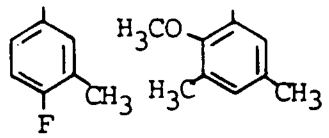 | | $-CH(CH_3)_2$ | $C_{22}H_{25}FO_2$ 340($M^+$) |
| 6 i | 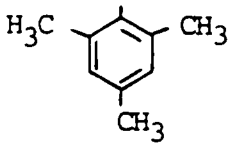 | 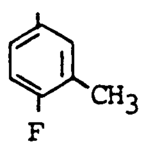 | $-CH(CH_3)_2$ | $C_{22}H_{25}FO$ 324($M^+$) |
| 6 j | 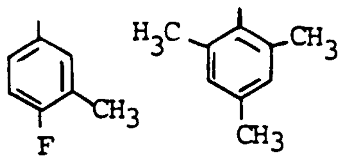 | | $-CH(CH_3)_2$ | $C_{22}H_{25}FO$ 324($M^+$) |
| 6 k | 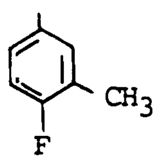 | 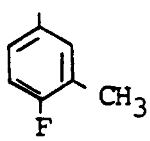 | $-CH(CH_3)_2$ | $C_{20}H_{20}F_2O$ 314($M^+$) |
| 6 l | 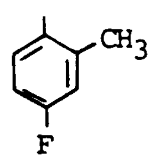 | 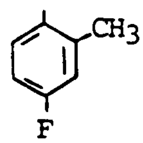 | $-CH(CH_3)_2$ | $C_{20}H_{20}F_2O$ 314($M^+$) |
| A-B gleich HC-CH | | | | |
| 6 m | 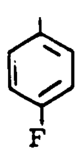 | 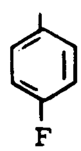 | 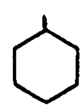 | $C_{21}H_{22}F_2O$, 328($M^+$),245,203 |

**Beispiel 7**
(Herstellung von Verbindungen der Formel (VI)

a) 4,4-Di-(4-fluorphenyl)-3-isopropylbutadienylcyanid

590 mg (12.3 mmol) Natriumhydrid wurden unter Argon vom Weißöl befreit und in 30 ml THF vorgelegt. Bei 0 °C wurden 2.0 ml (10.3 mmol) Cyanomethanphosphonsäurediisopropylester zugespritzt. Nach beendeter Gasentwicklung (ca. 30 min) wurden 2.52 g (8,8 mmol) der Verbindung gemäß Beispiel 6a in 50 ml THF eingetropft und 3h bei 10-15 °C gerührt. Die Mischung wurde auf gesättigte NaCl-Lösung gegossen und mit Ether extrahiert (3x). Die vereinigten organischen Phasen wurden einmal mit gesättigter NaCl-Lösung gewaschen und getrocknet ($MgSO_4$). Abziehen des Lösungsmittels im Vakuum ergab nach

Kristallisation aus Pentan 1.27 g 7a. Chromatographie der Mutterlauge auf Kieselgel (Cyclohexan/Essigester = 19:1) liefert weitere 1.09 g.
Ausbeute: 2.36 g (7,6 mmol, 86 %)
Schmp.: 108 ° C,
Rf (Cyclohexan/Essigester = 19:1) : 0.23
MS, $C_{20}H_{17}F_2N$: 309.1 ($M^+$)
$^1$H-NMR (60 MHz, $CDCl_3$): $\delta$ = 7.20-6.80 (m,9H), 5.40 (d, = 16 Hz,1H), 3.00 (sept.,1H), 1,08 (d,6H)

In Analogie zu Beispiel 7a wurden die Beispiele der Tabelle 7 erhalten

**Tabelle 7**

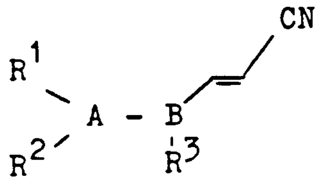

| Beispiel | $R^1$ | $R^2$ | $R^3$ | Massenspektrum |
|---|---|---|---|---|
| | | A-B gleich C=C | | |
| 7b | 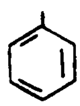 | 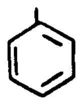 | $-CH_2CH(CH_3)_2$ | $C_{21}H_{21}N$, 287($M^+$) |

Fortsetzung Tabelle 7

| Beispiel | $R^1$ | $R^2$ | $R^3$ | Massenspektrum |
|---|---|---|---|---|
| 7b | 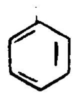 | 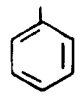 | $-CH_2CH(CH_3)_2$ | $C_{21}H_{21}N$, 287(M+) |
| 7 c | 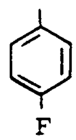 | 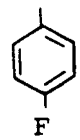 | $-CH_2CH(CH_3)_2$ | $C_{21}H_{19}F_2N$, 323(M+) |
| 7 d | 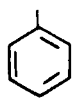 | 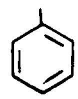 | 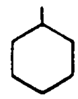 | $C_{23}H_{23}N$, 313(M+), 270,256 |
| 7 e | 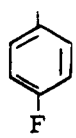 | 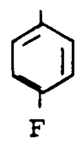 | $-(CH_2)_3CH_3$ | $C_{21}H_{19}F_2N$, 323(M+),280,266 |
| 7 f | 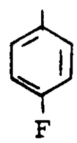 | 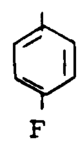 | 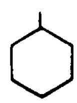 | $C_{23}H_{21}F_2N$, 349(M+) |
| 7 g | 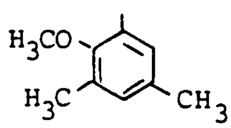 | 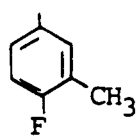 | $-CH(CH_3)_2$ | $C_{24}H_{26}FNO$, 363(M+) |
| 7 h | 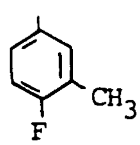 | 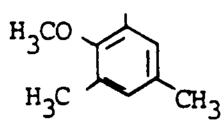 | $-CH(CH_3)_2$ | $C_{24}H_{26}FNO$, 363(M+), |
| 7 i | 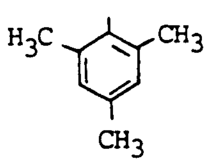 | 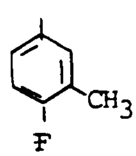 | $-CH(CH_3)_2$ | $C_{24}H_{26}FN$, 347(M+) |

Fortsetzung Tabelle 7

| Beispiel | $R^1$ | $R^2$ | $R^3$ | Massenspektrum |
|---|---|---|---|---|
| 7j | 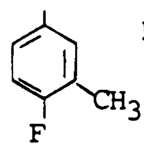 | 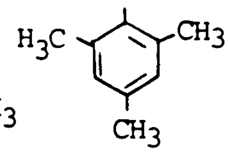 | $-CH(CH_3)_2$ | $C_{24}H_{26}FN$, 347($M^+$) |
| 7k | 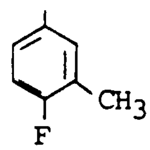 | 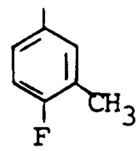 | $-CH(CH_3)_2$ | $C_{22}H_{21}F_2N$ 337($M^+$) |
| 7l | 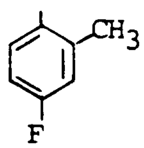 | 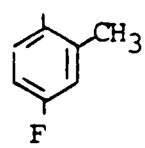 | $-CH(CH_3)_2$ | $C_{22}H_{21}F_2N$ 337($M^+$) |
| A-B gleich HC-CH | | | | |
| 7m | 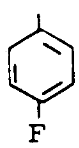 | 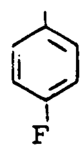 | 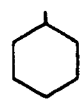 | $C_{23}H_{23}F_2N$ 351($M^+$) |

**Beispiel 8**
(Herstellung von Verbindungen der Formel III)

a) 5,5-Di-(4-fluorphenyl)-4-isopropylpentadienal

Zu 1.26 g (4.1 mmol) Beispiel 8a in 30 ml THF wurden bei 0°C 6.85 ml (8.2 mmol) Diisobutylaluminiumhydrid-Lösung (Toluol) gespritzt. Nach 4 h bei 10°C wurde vorsichtig mit 1n Salzsäure hydrolysiert und mit Ethylacetat extrahiert (3x). Die vereinigten organischen Phasen wurden einmal mit gesättigter Natriumhydrogencarbonatlösung gewaschen und getrocknet ($MgSO_4$). Abziehen des Lösungsmittels im Vakuum und FlashChromatographie auf Kieselgel (Cyclohexan/Essigester = 9:1 + 1% $NEt_3$) ergab 980 mg (3.14 mmol, 77%) Beispiel 8a.
Schmp. 80-82°C
Rf (Cyclohexan/Essigester = 5:1) : 0.38
MS, $C_{20}H_{18}F_2O$ : 312 ($M^+$), 297, 269
H-NMR: $\delta$ = 9.23 (d,J = 7Hz,1H), 7.30-6.70 (m,9H), 6.24 (dd, $J_1$ = 16Hz, $J_2$ = 7Hz,1H), 2.90 (sept., 1H), 1.08 (d,6H).

In Analogie zu Beispiel 8a wurden die Beispiele der Tabelle 8 erhalten

**Tabelle 8**

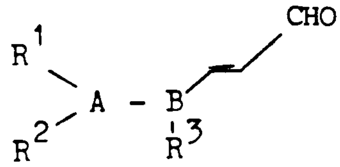

| Beispiel | $R^1$ | $R^2$ | $R^3$ | Massenspektrum |
|---|---|---|---|---|
| | | A-B gleich C=C | | |
| 8 b | 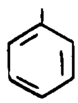 | 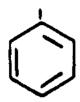 | $-CH_2CH(CH_3)_2$ | $C_{21}H_{22}O$, 290($M^+$), 247, 233, 205 |
| 8 c | 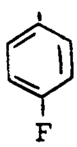 | 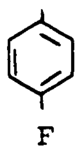 | $-CH_2CH(CH_3)_2$ | $C_{21}H_{20}F_2O$, 326($M^+$), 283,269 |
| 8 d | 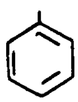 | 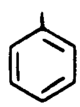 | 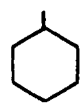 | $C_{23}H_{24}O$, 316($M^+$) |
| 8 e | 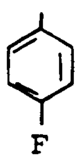 | 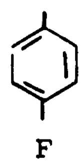 | $-(CH_2)_3CH_3$ | $C_{21}H_{20}F_2O$, 326($M^+$), 283,269 |

Fortsetzung Tabelle 8

| Beispiel | $R^1$ | $R^2$ | $R^3$ | Massenspektrum |
|---|---|---|---|---|
| 8 f | 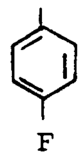 | 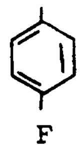 | 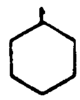 | $C_{23}H_{22}F_2O$, 352($M^+$), 349, 269 |
| 8 g | 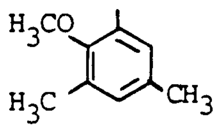 | 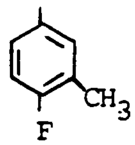 | $-CH(CH_3)_2$ | $C_{24}H_{27}FO_2$, 366($M^+$) |
| 8 h | 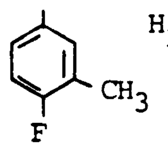 | 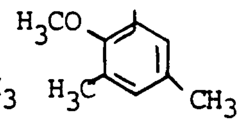 | $-CH(CH_3)_2$ | $C_{24}H_{27}FO_2$, 366($M^+$) |
| 8 i | 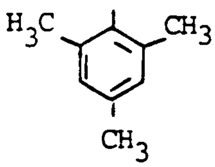 | 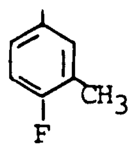 | $-CH(CH_3)_2$ | $C_{24}H_{27}FO$, 350($M^+$) |
| 8 j | 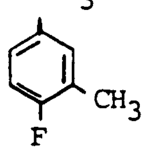 | 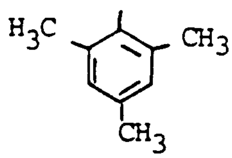 | $-CH(CH_3)_2$ | $C_{24}H_{27}FO$, 350($M^+$) |
| 8 k | 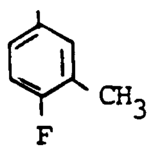 | 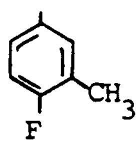 | $-CH(CH_3)_2$ | $C_{22}H_{22}F_2O$, 340($M^+$) |
| 8 l | 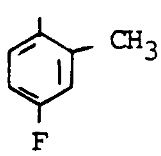 | 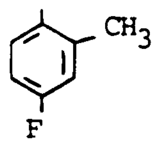 | $-CH(CH_3)_2$ | $C_{22}H_{22}F_2O$, 340($M^+$) |
| A-B gleich HC-CH | | | | |
| 8 m | 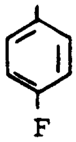 | 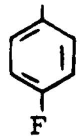 | 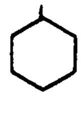 | $C_{23}H_{24}F_2O$, 354($M^+$) |

**Beispiel 9**
(Herstellung von Verbindungen der Formel IV)

a) 9,9-Di-(4-fluorphenyl)-5-hydroxy-3-oxo-8-isopropyl-6,8-nonadiensäureethylester

240 mg (5 mmol) vom Weißöl befreites Natriumhydrid wurden unter Argon in 15 ml trockenem THF vorgelegt. Bei 0 ° C spritzte man 597 μl (4.6 mmol) Acetessigsäureethylester zu und rührte 15 min bei 0 ° C. Anschließend wurden 2,8 ml (4.6 mmol) n-BuLi (Hexan) bei 0 ° C zugespritzt. Nach 15 min wurden bei -70 ° C 975 mg (3.1 mmol) der Verbindung gemäß Beispiel 8a in 40 ml THF zugetropft und 2.5h bei -70 ° C gerührt. Man ließ auf 0 ° C kommen, hydrolysierte mit kalter 2n Salzsäure und extrahierte mit Ether (3x). Die vereinigten organischen Phasen wurden einmal mit gesättigter Kochsalzlösung gewaschen und getrocknet ($MgSO_4$). Abziehen des Lösungsmittels ergab 1.41 g Beispiel 10a.

Rf (Cyclohexan/Essigester = 2:1):0.22

MS, $C_{26}H_{28}F_2O_4$ : 442 ($M^+$), 424

Die Verbindungen der Beispiele 9a - 9m wurden ohne weitere Reinigung und Charakterisierung zur nächsten Stufe eingesetzt.

In Analogie zu Beispiel 9a wurden die Beispiele der Tabelle 9 erhalten.

**Tabelle 9**

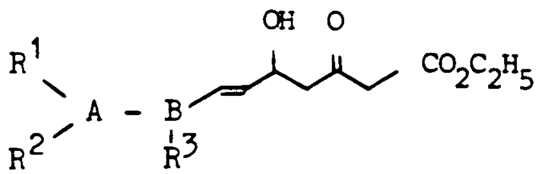

| Beispiel | $R^1$ | $R^2$ | $R^3$ | Molmasse |
|---|---|---|---|---|
| | | A-B gleich C=C | | |
| 9 b | 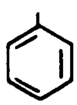 | 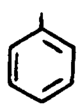 | $-CH_2CH(CH_3)_2$ | $C_{27}H_{32}O_4$, 420 |

Fortsetzung Tabelle 9

| Beispiel | $R^1$ | $R^2$ | $R^3$ | Molmasse |
|---|---|---|---|---|
| 9 c | 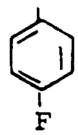 | 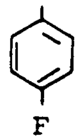 | $-CH_2CH(CH_3)_2$ | $C_{27}H_{30}F_2O_4$, 456 |
| 9 d | 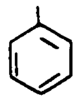 | 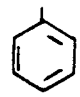 | 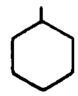 | $C_{29}H_{34}O_4$, 446 |
| 9 e | 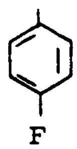 | 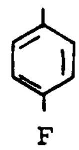 | $-(CH_2)_3CH_3$ | $C_{27}H_{30}F_2O_4$, 456 |
| 9 f | 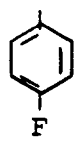 | 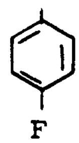 | 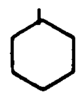 | $C_{29}H_{32}F_2O_4$, 482 |
| 9 g | 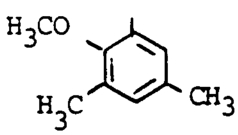 | 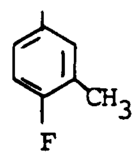 | $-CH(CH_3)_2$ | $C_{30}H_{37}FO_5$, 496 |
| 9 h | 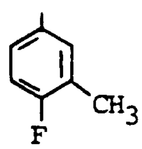 | 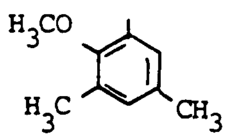 | $-CH(CH_3)_2$ | $C_{30}H_{37}FO_5$, 496 |
| 9 i | 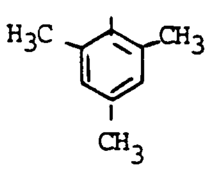 | 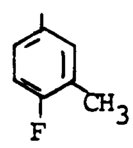 | $-CH(CH_3)_2$ | $C_{30}H_{37}FO_4$, 480 |
| 9 j | 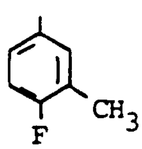 | 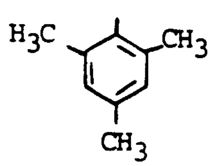 | $-CH(CH_3)_2$ | $C_{30}H_{37}FO_4$, 480 |

**Fortsetzung Tabelle 9**

| Beispiel | $R^1$ | $R^2$ | $R^3$ | Molmasse |
|---|---|---|---|---|
| 9 k | 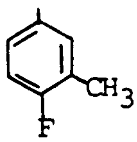 | 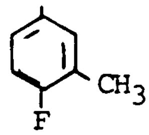 | $-CH(CH_3)_2$ | $C_{28}H_{32}F_2O_4$, 470 |
| 9 l | 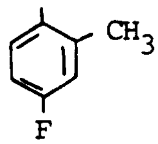 | 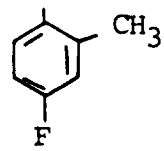 | $-CH(CH_3)_2$ | $C_{28}H_{32}F_2O_4$, 470 |
| | | A-B gleich HC-CH | | |
| 9 m | 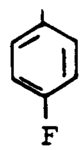 | 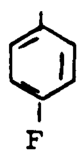 | 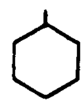 | $C_{29}H_{34}F_2O_4$, 484 |

**Beispiel 10**
(Herstellung von Verbindungen der Formel I)

a) 9,9-Di-(4-fluorphenyl)-3,5-dihydroxy-8-isopropyl-6,8-nonadiensäureethylester

1.4 g (3.1 mmol) der Verbindung gemäß Beispiel 9a wurden unter Argon in 20 ml trockenem THF mit 5.0 ml (5 mmol) Triethylboranlösung (THF) versetzt und 10 min bei Raumtemperatur gerührt. Bei -70 °C wurden 240 mg (6.2 mmol) Natriumborhydrid fest zugegeben. Anschließend spritzte man langsam 3 ml Methanol zu. Nach 3h bei -70 °C groß man die Mischung vorsichtig auf eine gekühlte Lösung von 5 ml 35 proz. Wasserstoffperoxid in 100 ml Wasser. Nach 5 min wurde mit Essigester extrahiert (3x). Die vereinigten organischen Phasen wurden mit gesättigter Natriumhydrogencarbonatlösung (2x) und gesättigter Kochsalzlösung (1x) gewaschen und getrocknet ($MgSO_4$). Abziehen des Lösungsmittels im Vakuum und Flash-Chromatographie auf Kieselgel (Cyclohexan/Essigester = 3:2 + 0,5% $Et_3N$) ergab 720 mg (1,62 mmol, 52% über zwei Stufen) Beispiel 10a
Rf (Cyclohexan/Essigester = 2:1) : 0.11
MS, $C_{26}H_{30}F_2O_4$ : 444 ($M^+$) 426,408
H-NMR (270 MHz, $CDCl_3$) : $\delta$ = 7.10-6.80 (m8H), 6.15 (d, J = 16Hz, 1H), 5.53 (dd,$J_1$ = 16Hz,$J_2$ = 7,2 Hz, 1H), 4,30 (m,1H), 4,17 (q,2H), 4.10 (m, 1H) 2,83 (sept.,1H), 2,43 (m,2H), 1,52 (m,1H) 1,37 (m, 1H) 1,28 (t,3H), 1.09 (d, 6H).

Tabelle 10

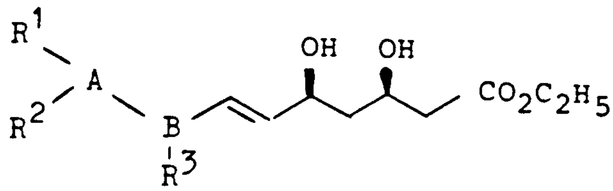

| Beispiel | $R^1$ | $R^2$ | $R^3$ | Charakteristische analytische Daten |
|---|---|---|---|---|
| | | A-B gleich C=C | | |
| 10b | 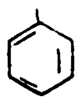 | 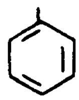 | $-CH_2CH(CH_3)_2$ | MS, $C_{27}H_{34}O_4$: 422($M^+$),404,387 H-NMR (270 MHz, $CDCl_3$), δ= 7.35-7.05 (m,10H), 6.40 (d, $J_1$=16Hz,1H), 5.75 (dd, $J_1$=16Hz, $J_2$= 8Hz,1H), 4,33 (m,1H), 4.20 (m,1H), 4.15 (q,2H), 2.43 (m,2H), 2.24 (d,2H), 1,85 (m,1H), 1.70 (m,1H), 1.65 (m,1H), 1.24 (t,3H), 0.73 (d,6H). |
| 10c | 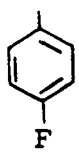 | 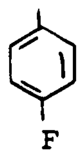 | $-CH_2CH(CH_3)_2$ | MS, $C_{27}H_{32}F_2O_4$: 458($M^+$),441,423 H-NMR (270 MHz), $CDCl_3$): δ= 7.15-6,92 (m,8H), 6.48 (d, $J_1$= 16Hz,1H), 5.79 (dd, $J_1$=16Hz, $J_2$= 7.6Hz,1H), 4.39 (m,1H), 4.24 (m,1H), 4.19 (q,2H), 3.64 und 3.07 (breit jeweils 1H), 2.49 (m,2H), 2.27 (d,2H), 1.88 (m,1H), 1,72 (m,1H), 1.50 (m,1H), 1.29 (t,3H), 0.78 (d,6H) |

Fortsetzung Tabelle 10

| Beispiel | $R^1$ | $R^2$ | $R^3$ | Charakteristische analytische Daten |
|---|---|---|---|---|
| 10d | 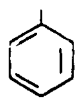 | 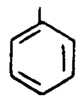 | 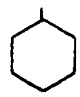 | MS, $C_{29}H_{36}O_4$: 448($M^+$),431,413 H-NMR (270 MHz), $CDCl_3$): δ= 7.35-7.00 (m,10H), 6.17 (d, $J_1$=16Hz,1H), 5.46 (dd,$J_1$=16Hz, $J_2$= 7.2Hz,1H), 4.23 (m,1H), 4.18 (q,2H), 4.06 (m,1H), 2.55-2.31 (m,3H), 1.75-1.40 1.35-1.20 und 1.10 (jeweils m, zusammen 15H). |
| 10e | 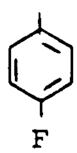 | 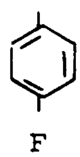 | $-(CH_2)_3CH_3$ | MS, $C_{27}H_{32}F_2O_4$: 458($M^+$),440,422 H-NMR (270 MHz, $CDCl_3$): δ= 7.10-6.90 (m,8H), 6.31 (d, $J_1$=16Hz,1H), 5.80 (dd,$J_1$= 16Hz, $J_2$=6.4Hz,1H), 4.40 (m,1H), 4.25 (m,1H), 4.18 (q,2H), 2.48 (m,2H), 2.27 (t,2H), 1.72 (m,1H), 1.60 (m,1H), 1.45 (m,2H), 1.28 (t,3H), 1.25 (m,2H), 0.82 (t,3H). |
| 10f | 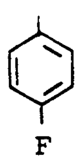 | 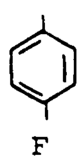 | 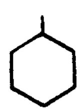 | MS, $C_{29}H_{34}F_2O_4$: 484($M^+$),466 H-NMR (270 MHz, $CDCl_3$): δ= 7.15-6.80 (m,8H), 6.16 (d, $J_1$ =16Hz,1H), 5.48 (dd,$J_1$= 16Hz, $J_2$ = 7.4Hz,1H), 4.28 (m,1H), 4.18 (q,2H), 4.10 (m,1H), 2.53-2,34 (m,3H), 1.80-1.00 (m,15H) |

Fortsetzung Tabelle 10

| Beispiel | $R^1$ | $R^2$ | $R^3$ | Charakteristische analytische Daten |
|---|---|---|---|---|
| 10 g | 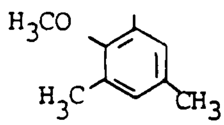 | 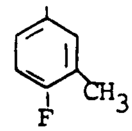 | $-CH(CH_3)_2$ | MS, $C_{30}H_{39}FO_5$: 498 ($M^+$), 480<br>DC (Dichlormethan/Methanol= 4:1)<br>Rf = 0.40 |
| 10 h | 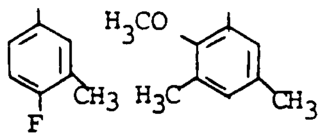 | | $-CH(CH_3)_2$ | MS, $C_{30}H_{39}FO_5$: 498 ($M^+$), 480<br>DC (Dichlormethan/Methanol= 4:1):<br>Rf = 0.40 |
| 10 i | 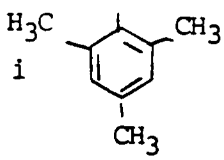 | 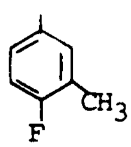 | $-CH(CH_3)_2$ | MS, $C_{30}H_{39}FO_4$: 482 ($M^+$), 464<br>DC (Dichlormethan/Methanol= 5:1):<br>Rf = 0.32 |
| 10 j | 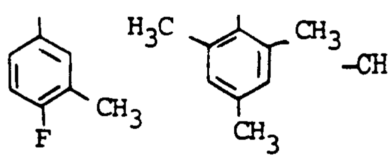 | | $-CH(CH_3)_2$ | MS, $C_{30}H_{39}FO_4$: 482 ($M^+$), 464<br>DC (Dichlormethan/Methanol= 5:1)<br>Rf = 0.32 |

Fortsetzung Tabelle 10

| Beispiel | $R^1$ | $R^2$ | $R^3$ | Charakteristische analytische Daten |
|---|---|---|---|---|
| 10k | 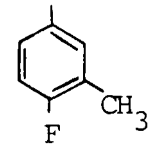 | 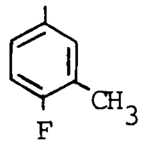 | $-CH(CH_3)_2$ | MS, $C_{28}H_{34}F_2O_4$: 472 ($M^+$), 454<br>DC (Dichlormethan/Methanol= 4:1):<br>Rf= 0.39 |
| 10l | 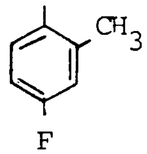 | 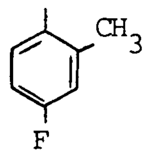 | $-CH(CH_3)_2$ | MS, $C_{28}H_{34}F_2O_4$: 472 ($M^+$), 454<br>DC (Dichlormethan/Methanol= 4:1):<br>Rf= 0.39 |
| | | A-B gleich | | |
| 10m | 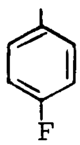 | 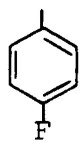 | 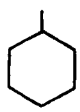 | MS, $C_{29}H_{36}F_2O_4$: 486 ($M^+$), 468<br>DC (Dichlormethan/Methanol= 4:1):<br>Rf= 0.42 |

Beispiel 10n
(Herstellung einer Verbindung der Formel I, A-B gleich C=C, X-Y gleich $H_2C-CH_2$)

n) 3,5-Dihydroxy-9,9-di(4-flurophenyl)-8-isopropyl-8-nonensäureethylester

240 mg (0.55 mmol) der Verbindung gemäß Beispiel 10a wurden in 10 ml Ethanol mit 100 mg Palladium auf Kohle (10%) bei Raumtemperatur und Normaldruck hydriert. Nach 2h war die $H_2$-Aufnahme beendet und es wurde vom Katalysator abfiltriert und eingeengt. Chromatographie auf Kieselgel (Cyclohexan/Essigester= 2:1) ergab 100 mg Beispiel 10n. MS (FAB), $C_{26}H_{32}F_2O_4$: 469 (M + $Na^+$), 447,429
H-NMR (270 MHz,$CDCl_3$): $\delta$= 7.15-6.90(m,8H), 4.20-4.05(m,3H), 3.60(m,1H) 2.76(sept.,1H), 2.40(m,2H), 2.26-2.00(m,2H), 1.56-1.16(m,7H), 1.01 (dd,6H)

**Beispiel 11**
(Herstellung von Verbindungen der Formel I)

a) 9,9-Di-(4-fluorphenyl)-3,5-dihydroxy-8-isopropyl-6,8-nonadiensäure Natriumsalz

445 mg (1.0 mmol) der Verbindung gemäß Beispiel 10a wurden in 20 ml Ethanol gelöst und mit 10 ml (1 mmol) 0.1n Natronlauge versetzt. Nach 1h bei Raumtemperatur wurde das Lösungsmittel unter mehrmaligem Zusatz von Toluol im Vakuum abgezogen. Der feste Rückstand wurde mehrmals mit n-Pentan gewaschen und im Hochvakuum getrocknet.
Rf ($CHCl_3$/MeOH= 7:5):0.45
H-NMR (270 MHz, $D_2O$): $\delta$= 7.0-6.7 (m,8H), 6.04 (d, $J_1$=16Hz,1H), 5.38 (dd, $J_1$= 16Hz, $J_2$=8Hz,1H), 4.02 (m,1H), 3.65 (m,1H), 2.61 (sept., 1H), 2.16 (m,2H), 1.50 (m,1H), 1.25 (m,1H), 0.90 (d,2H)
In Analogie zu Beispiel 11a wurden die Beispiele der Tabelle 11 erhalten.

**Tabelle 11**

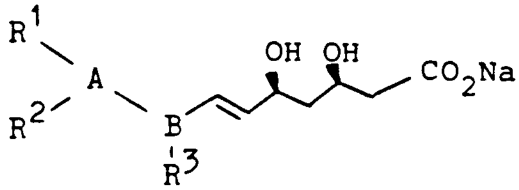

| Beispiel | $R^1$ | $R^2$ | $R^3$ | Molmasse |
|---|---|---|---|---|
| | | A-B gleich C=C | | |
| 11b | 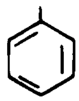 | 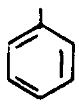 | $-CH_2CH(CH_3)_2$ | $C_{25}H_{29}O_4Na$,416.5 |
| 11c | 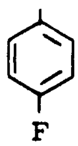 | 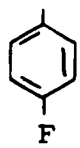 | $-CH_2CH(CH_3)_2$ | $C_{25}H_{27}F_2O_4Na$,452.4 |
| 11d | 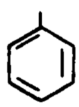 | 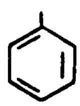 | 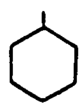 | $C_{27}H_{31}O_4Na$,442.5 |

Fortsetzung Tabelle 11

| Beispiel | $R^1$ | $R^2$ | $R^3$ | Molmasse |
|---|---|---|---|---|
| | | A-B gleich C=C | | |
| 11e | 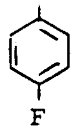 | 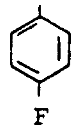 | $-(CH_2)_3CH_3$ | $C_{25}H_{27}F_2O_4Na$, 452.4 |
| 11f | 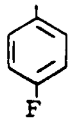 | 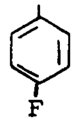 | 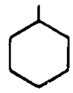 | $C_{27}H_{29}F_2O_4Na$, 478.5 |
| 11g | 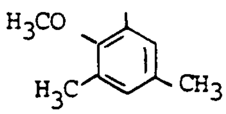 | 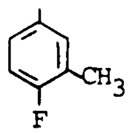 | $-CH(CH_3)_2$ | $C_{28}H_{34}FO_5Na$, 492.5 |
| 11h | 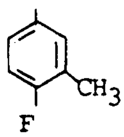 | 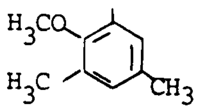 | $-CH(CH_3)_2$ | $C_{28}H_{34}FO_5Na$, 492.5 |
| 11i | 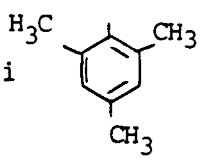 | 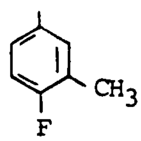 | $-CH(CH_3)_2$ | $C_{28}H_{34}FO_4Na$, 476.5 |
| 11j | 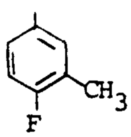 | 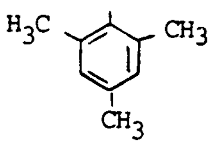 | $-CH(CH_3)_2$ | $C_{28}H_{34}FO_4Na$, 476.5 |
| 11k | 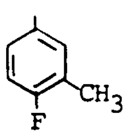 | 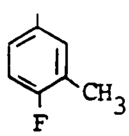 | $-CH(CH_3)_2$ | $C_{26}H_{29}F_2O_4Na$, 466.5 |
| 11l | 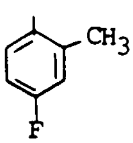 | 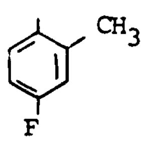 | $-CH(CH_3)_2$ | $C_{26}H_{29}F_2O_4Na$, 466.5 |

Fortsetzung Tabelle 11

| Beispiel | $R^1$ | $R^2$ | $R^3$ | Molmasse |
|---|---|---|---|---|
| | | A-B gleich HC-CH | | |
| 11m | 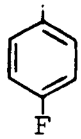 | 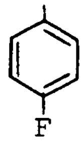 | 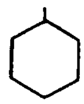 | $C_{27}H_{31}F_2O_4Na$, 480.5 |

**Beispiel 11n**

In analoger Weise wie in Beispiel 11a beschrieben, erhält man aus der Verbindung gemäß Beispiel 10n
3,5-Dihydroxy-9,9-di-(4-flurophenyl)-8-isopropyl-8-nonensäure-Natriumsalz
Verbindung der Formel I, A-B gleich C=C, X-Y gleich $H_2C$-$CH_2$ $R^1$ = $R^2$ = 4-fluorphenyl, $R^3$ = isopropyl, $R^4$ = Na $C_{24}H_{27}F_2O_4Na$, 440.4
Synthese der optisch aktiven Verbindung entsprechend Beispiel 11a

**Beispiel 12**
(3S, (1′S)-7,7-Di-(4-fluorphenyl)-3-hydroxy-6-isopropyl 4,6-heptadiensäure-1',2',2'-triphenyl-1′ ,2′-ethandiol-1′-ester

Zu einer aus 506 µl (3.6 mmol) Diisopropylamin und 2.26 ml N-BuLi (Hexan) in 5 ml THF bei 0 °C bereiteten LDA-Lösung gab man bei -78 °C unter Argon 340 mg (1.02 mmol) (S)-1,2,2-Triphenyl-1,2-ethandiol-1-acetat (Tetrahedron Lett. 25, 5031 (1984)). Man ließ auf -20 °C kommen bis sich alles gelöst hatte und kühlte wieder auf -78 °C. Zu dieser Lösung tropfte man 318 mg (1.02 mmol) Beispiel 8a in wenig THF. Nach 2 h bei -78 °C wurde auf gesättigte wäßrige Ammoniumchloridlösung gegossen und mit Ether extrahiert (3x). Die vereinigten organischen Phasen wurden getrocknet ($MgSO_4$) und das Lösungsmittel entfernt. Der Rückstand wurde auf Kieselgel chromatographiert (Cyclohexan/Essigester = 2:1 + 1 % $NEt_3$). Eine weitere Auftrennung der Diastereomeren gelingt durch HPLC. DC (Cyclohexan/Essigester = 3:1): Rf = 0.13

**Beispiel 13**
(3S)-7,7-Di-(4-fluorphenyl)-3-hydroxy-6-isopropyl-4,6-heptadiensäuremethylester

77 mg (0.12 mmol) Hydroxyester gemäß Beispiel 12 wurden in 13 ml Methanol gelöst und mit einer Lösung von 2,7 mg (0.12 mmol) Natrium in 1.35 ml Methanol versetzt und bei Raumtemperatur gerührt. Nach beendeter Umsetzung wurde das Lösungsmittel in Vakuum entfernt und der Rückstand auf Kieselgel chromatographiert (Cyclohexan/Essigester = 3:1 + 1 % $NEt_3$).
MS, $C_{23}H_{24}F_2O_3$: 386, 369

**Beispiel 14**
(5S)-9,9-Di-(4-fluorphenyl)-5-hydroxy-3-oxo-8-isopropyl-6,8-nonadiensäure-t-butylester

Zu einer LDA-Lösung, bereitet aus 0.260 ml (1.86 mmol) Diisopropylamin und 1.16 ml n-BuLi (Hexan) in 3 ml THF tropfte man bei -78 °C unter Argon 250 µl (1.86 mmol) Essigsäure-t-butylester. Nach 30 min bei -78 °C tropfte man 120 mg (0.310 mmol) der Verbindung gemäß Beispiel 13 in wenig THF zu und ließ 3 h zwischen -30 °C und -40 °C rühren. Die Reaktionslösung wurde auf gesättigte wäßrige Ammoniumchloridlösung gegossen und mit Ether extrahiert (3x). Trocknen mit Magnesiumsulfat und Abziehen des Lösungsmittel gab Beispiel 14, das nicht weiter gereinigt wurde.

DC (Cyclohexan/Essigester = 3:1): mehrmals entwickelt.

**Beispiel 15**

(3R),(5S)-9,9-Di(4-fluorphenyl)-3,5-dihydroxy-8-isopropyl-6,8-nonadiensäure-t-butylester

In Analogie zu Beispiel 10a wurde die Verbindung gemäß Beispiel 15 aus der Verbindung gemäß Beispiel 14 erhalten.
DC (Cyclohexan/Essigester = 2:1): Rf = 0.23 (15),
Rf = 0.39 (14).
MS, $C_{28}H_{34}O_4F_2$: 472 ($M^+$), 455, 437

**Beispiel 16**

(3R),(5S)-9,9-Di-(4-fluorphenyl)-3,5-dihydroxy-8-isopropyl-6,8-nonadiensäure Natriumsalz (optisch aktives 11a)

In Analogie zu Beispiel 11a wurde optisch aktives 11a aus der Verbindung gemäß Beispiel 15 erhalten.

**Patentansprüche**

**Patentansprüche für folgende Vertragsstaaten : AT, BE, CH, DE, FR, GB, IT, LI, LU, NL, SE**

**1.** 3,5-Dihydroxycarbonsäuren und deren Derivate der allgemeinen Formel I

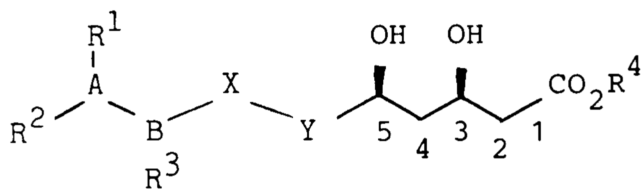

I

und der entsprechenden Laktone der Formel II

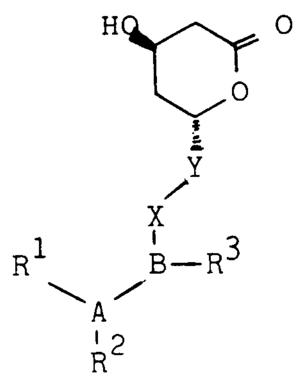

II

wobei in den allgemeinen Formeln I und II bedeuten

A-B CH-CH oder C = C,
X-Y $CH_2$-$CH_2$ oder HC = CH,

$R^1$ und $R^2$, wobei $R^1$ und $R^2$ gleich oder verschieden sind, einen gesättigten oder ungesättigten Alkylrest mit bis zu 20 C-Atomen, der mit einer Alkoxygruppe mit 1-6 C-Atomen oder der Gruppe

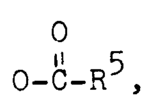

wobei $R^5$ Alkyl mit 1-8 C-Atomen bedeutet, substituiert sein kann, einen Cycloalkylrest mit 3-7 C-Atomen, einen Phenyl-, Thiophen-, Furan- oder Naphtalinrest, wobei die aromatischen Reste im Kern 1-3 fach substituiert sein können mit Halogen, Alkyl- oder Alkoxy mit jeweils 1-6 C-Atomen, Cycloalkyl mit 3-7 C-Atomen oder der Gruppe

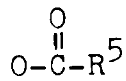

wobei $R^5$ Alkyl mit 1-8 C-Atomen bedeutet, oder einen Pyridinrest, der im Kern 1-3 fach substituiert sein kann mit Alkyl mit 1-4 C-Atomen,

$R^3$ einen gesättigten oder ungesättigten Alkylrest mit bis zu 8 C-Atomen, einen Benzylrest, welcher im Kern 1-3 fach substituiert sein kann mit Halogen, Alkoxy oder Alkyl mit jeweils 1-4 C-Atomen, einen Phenyl-, Thiophen-, Furan-, Naphtalinrest, wobei die aromatischen Reste im Kern 1-3 fach substituiert sein können mit Halogen, Alkoxy oder Alkyl mit jeweils 1-4 C-Atomen, oder einen Cycloalkylrest mit 3-7 C-Atomen

$R^4$ in Formel I Wasserstoff, Alkyl mit 1-8 C-Atomen, ein Metallkation, ein Ammoniumion, Phenyl, Benzyl oder den 2,3-Dihydroxypropylrest.

**2.** Verbindungen der allgemeinen Formeln I und II gemäß Anspruch 1, dadurch gekennzeichnet, daß

A-B CH-CH oder C = C
X-Y $CH_2$-$CH_2$ oder HC = CH

$R^1$ und $R^2$, wobei $R^1$ und $R^2$ gleich oder verschieden sind, einen Alkylrest mit 1-10 C-Atomen, der mit einer Alkoxygruppen mit 1-6 C-Atomen oder der Gruppe

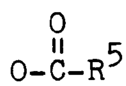,

wobei $R^5$ Alkyl mit 1-8 C-Atomen bedeutet, substituiert sein kann, einen Cycloalkylrest mit 5-6 C-Atomen, einen Phenylrest welcher im Kern 1-3 fach substituiert sein kann mit Halogen, Alkyl oder Alkoxy mit jeweils 1-4 C-Atomen, Cycloalkyl mit 5-6 C-Atomen, oder der Gruppe

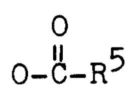,

wobei
$R^5$ Alkyl mit 1-8 C-Atomen bedeutet,

$R^3$ einen Alkylrest mit 1-8 C-Atomen, einen Benzylrest, welcher im Kern 1-3 fach substituiert sein kann mit Halogen, Alkoxy oder Alkyl mit jeweils 1-4 C-Atomen, einen Phenylrest welcher im Kern 1-3 fach substituiert sein kann mit Halogen, Alkoxy oder Alkyl mit jeweils 1-4 C-Atomen, oder einen Cycloalkylrest mit 5-6 C-Atomen,

$R^4$ in Formel I Wasserstoff, Alkyl mit 1-6 C-Atomen, ein Metallkation, ein Ammoniumion, Phenyl oder Benzyl bedeuten.

**3.** Verbindungen der allgemeinen Formeln I und II gemäß Anspruch 1, dadurch gekennzeichnet, daß

A-B C = C,
X-Y HC = CH,

$R^1$ und $R^2$, wobei $R^1$ und $R^2$ gleich oder verschieden sind, einen Phenylrest, welcher im Kern 1-3 fach substituiert sein kann mit Halogen, Alkyl oder Alkoxy mit jeweils 1 bis 4 C-Atomen oder der Gruppe

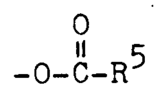

wobei $R^5$ Alkyl mit 1-8 C-Atomen bedeutet,

$R^3$ einen Alkylrest mit 1-8 C-Atomen oder einen Cycloalkylrest mit 5-6 C-Atomen,

$R^4$ in Formel I Wasserstoff, Alkyl mit 1-8 C-Atomen, ein Natrium- oder Kaliumkation oder ein Ammoniumion, bedeuten.

**4.** Verbindungen gemäß Anspruch 1, dadurch gekennzeichnet, daß sie die Formel I besitzen.

**5.** Verfahren zur Herstellung der Verbindungen der allgemeinen Formeln I und II gemäß Anspruch 1, dadurch gekennzeichnet ist, daß man
a) entsprechend substituierte Aldehyde der Formel III

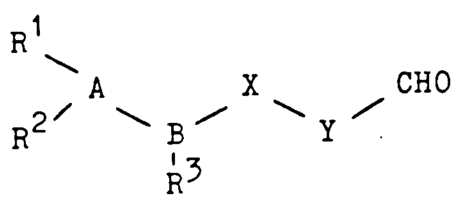

III

worin A-B, X-Y, $R^1$,$R^2$ und $R^3$ die angegebenen Bedeutungen haben, in die entsprechenden Hydroxyketoester der allgemeinen Formel IV überführt

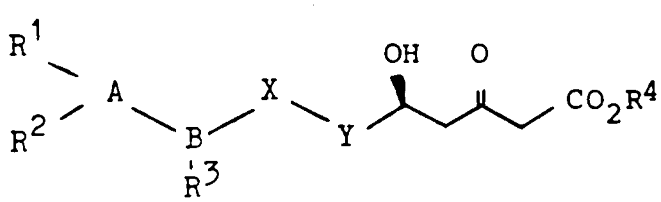

IV

worin A-B, X-Y, $R^1$,$R^2$ und $R^3$ die angegebenen Bedeutungen haben und $R^4$ Alkyl mit 1-8 C-Atomen bedeutet,
b) die Hydroxyketoester der Formel IV in die entsprechenden 3,5-Dihydroxyverbindungen der Formel I

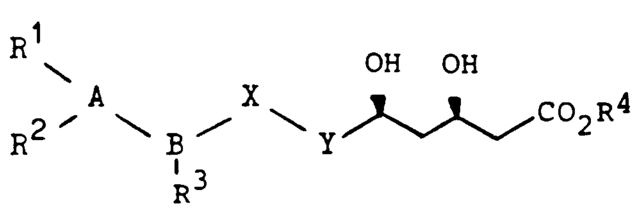

I

worin A-B, X-Y, $R^1$,$R^2$ und $R^3$ die zu Formel I angegebenen Bedeutungen haben, und $R^4$ Alkyl mit 1-8 C-Atomen ist, überführt und eine erhaltene Verbindung gegebenenfalls verseift zu einer Verbindung der Formel I, worin $R^4$ ein Metallkation darstellt, daraus gegebenenfalls die freie Säure ($R^4$ = Wasserstoff) in Freiheit setzt und die freie Säure gegebenenfalls in Verbindungen der Formel I mit

$R^4$ = Ammoniumion, Phenyl, Benzyl oder 2,3-Dihydroxypropyl überführt,
c) und eine erhaltene Verbindung der Formel I gegebenenfalls in ein Lakton der Formel II überführt,

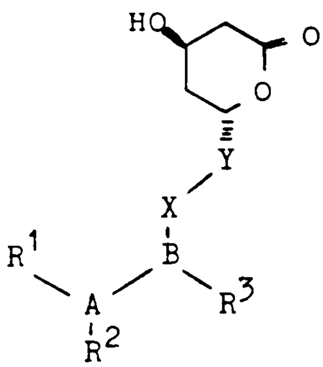

II

worin A-B, X-Y $R^1$,$R^2$ und $R^3$ die angegebenen Bedeutungen haben.

6. Pharmazeutische Präparate enthaltend eine Verbindung gemäß Anspruch 1.

7. Verbindung gemäß Anspruch 1 zur Prophylaxe und Therapie der Hypercholesterinämie.

8. Verbindungen der Formel IV

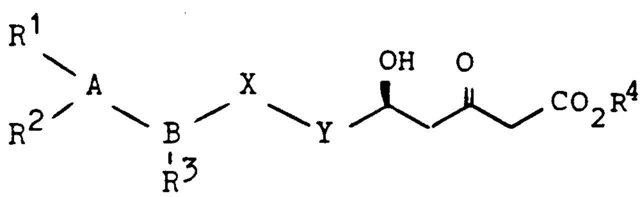

worin A-B, X-Y, $R^1$, $R^2$ und $R^3$ die in Anspruch 1 zu Formel I angegebene Bedeutung haben und $R^4$ Alkyl mit 1-8 C-Atomen ist.

**Patentansprüche für folgende Vertragsstaaten : ES, GR,**

1. Verfahren zur Herstellung von 3,5-Dihydroxycarbonsäuren und deren Derivaten der allgemeinen Formel I

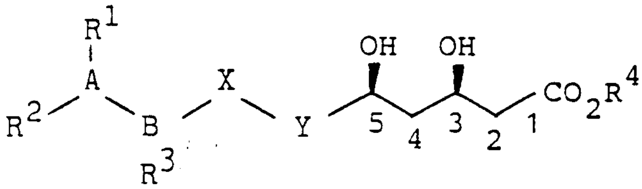

I

und der entsprechenden Laktone der Formel II

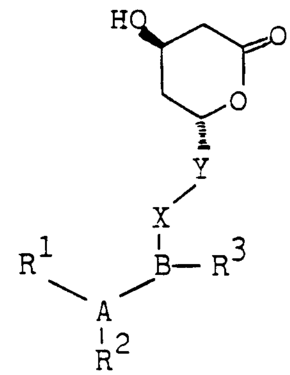

II

wobei in den allgemeinen Formeln I und II bedeuten

A-B CH-CH oder C = C,
X-Y $CH_2$-$CH_2$ oder HC = CH,

$R^1$ und $R^2$, wobei $R^1$ und $R^2$ gleich oder verschieden sind, einen gesättigten oder ungesättigten Alkylrest mit bis zu 20 C-Atomen, der mit einer Alkoxygruppe mit 1-6 C-Atomen oder der Gruppe

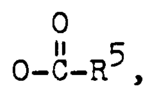

wobei $R^5$ Alkyl mit 1-8 C-Atomen bedeutet, substituiert sein kann, einen Cycloalkylrest mit 3-7 C-Atomen, einen Phenyl-, Thiophen-, Furan- oder Naphtalinrest, wobei die aromatischen Reste im Kern 1-3 fach substituiert sein können mit Halogen, Alkyl- oder Alkoxy mit jeweils 1-6 C-Atomen, Cycloalkyl mit 3-7 C-Atomen oder der Gruppe

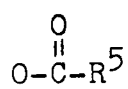

wobei $R^5$ Alkyl mit 1-8 C-Atomen bedeutet, oder einen Pyridinrest, der im Kern 1-3 fach substituiert sein kann mit Alkyl mit 1-4 C-Atomen,

$R^3$ einen gesättigten oder ungesättigten Alkylrest mit bis zu 8 C-Atomen, einen Benzylrest, welcher im Kern 1-3 fach substituiert sein kann mit Halogen, Alkoxy oder Alkyl mit jeweils 1-4 C-Atomen, einen Phenyl-, Thiophen-, Furan-, Naphtalinrest, wobei die aromatischen Reste im Kern 1-3 fach substituiert sein können mit Halogen, Alkoxy oder Alkyl mit jeweils 1-4 C-Atomen, oder einen Cycloalkylrest mit 3-7 C-Atomen,

$R^4$ in Formel I Wasserstoff, Alkyl mit 1-8 C-Atomen, ein Metallkation, ein Ammoniumion, Phenyl, Benzyl oder den 2,3-Dihydroxypropylrest,
dadurch gekennzeichnet ist, daß man
a) entsprechend substituierte Aldehyde der Formel III

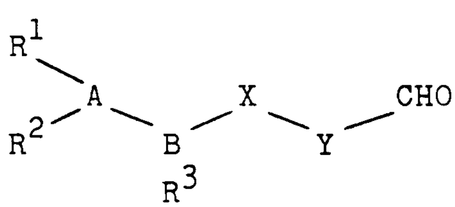

III

worin A-B, X-Y, $R^1$,$R^2$ und $R^3$ die angegebenen Bedeutungen haben, in die entsprechenden Hydroxyketoester der allgemeinen Formel IV überführt

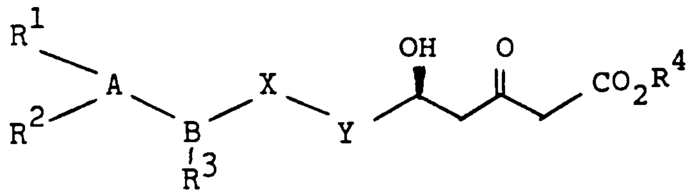

IV

worin A-B, X-Y, $R^1$,$R^2$ und $R^3$ die angegebenen Bedeutungen haben und $R^4$ Alkyl mit 1-8 C-Atomen bedeutet,

b) die Hydroxyketoester der Formel IV in die entsprechenden 3,5-Dihydroxyverbindungen der Formel I

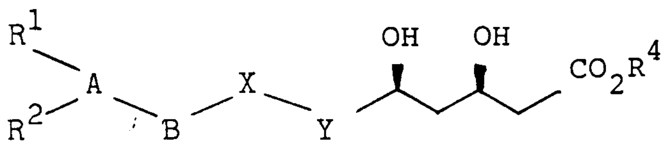

I

worin A-B, X-Y, $R^1$,$R^2$ und $R^3$ die zu Formel I angegebenen Bedeutungen haben, und $R^4$ Alkyl mit 1-8 C-Atomen ist, überführt und eine erhaltene Verbindung gegebenenfalls verseift zu einer Verbindung der Formel I, worin $R^4$ ein Metallkation darstellt, daraus gegebenenfalls die freie Säure ($R^4$ = Wasserstoff) in Freiheit setzt und die freie Säure gegebenenfalls in Verbindungen der Formel I mit $R^4$ = Ammoniumion, Phenyl, Benzyl oder 2,3-Dihydroxypropyl überführt,

c) und eine erhaltene Verbindung der Formel I gegebenenfalls in ein Lakton der Formel II überführt,

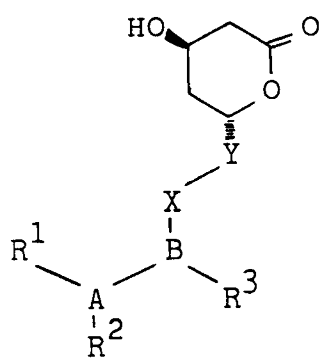

II

worin A-B, X-Y, $R^1$,$R^2$ und $R^3$ die angegebenen Bedeutungen haben.

**2.** Verfahren gemäß Anspruch 1, dadurch gekennzeichnet, daß man eine Verbindung der allgemeinen Formel I oder II, worin

A-B CH-CH oder C=C
X-Y $CH_2$-$CH_2$ oder HC=CH

$R^1$ und $R^2$, wobei $R^1$ und $R^2$ gleich oder verschieden sind, einen Alkylrest mit 1-10 C-Atomen, der mit einer Alkoxygruppen mit 1-6 C-Atomen oder der Gruppe

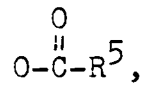

wobei $R^5$ Alkyl mit 1-8 C-Atomen bedeutet, substituiert sein kann, einen Cycloalkylrest mit 5-6 C-Atomen, einen Phenylrest welcher im Kern 1-3 fach substituiert sein kann mit Halogen, Alkyl oder Alkoxy mit jeweils 1-4 C-Atomen, Cycloalkyl mit 5-6 C-Atomen, oder der Gruppe

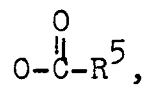

wobei
$R^5$ Alkyl mit 1-8 C-Atomen bedeutet,

$R^3$ einen Alkylrest mit 1-8 C-Atomen, einen Benzylrest, welcher im Kern 1-3 fach substituiert sein kann mit Halogen, Alkoxy oder Alkyl mit jeweils 1-4 C-Atomen, einen Phenylrest welcher im Kern 1-3 fach substituiert sein kann mit Halogen, Alkoxy oder Alkyl mit jeweils 1-4 C-Atomen, oder einen Cycloalkylrest mit 5-6 C-Atomen,

$R^4$ in Formel I Wasserstoff, Alkyl mit 1-6 C-Atomen, ein Metallkation, ein Ammoniumion, Phenyl oder Benzyl bedeuten, herstellt.

**3.** Verfahren gemäß Anspruch 1, dadurch gekennzeichnet, daß man eine Verbindung der allgemeinen Formel I oder II, worin

A-B C = C,
X-Y HC = CH,

$R^1$ und $R^2$, wobei $R^1$ und $R^2$ gleich oder verschieden sind, einen Phenylrest, welcher im Kern 1-3 fach substituiert sein kann mit Halogen, Alkyl oder Alkoxy mit jeweils 1 bis 4 C-Atomen oder der Gruppe

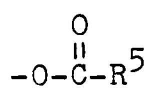

wobei $R^5$ Alkyl mit 1-8 C-Atomen bedeutet,

$R^3$ einen Alkylrest mit 1-8 C-Atomen oder einen Cycloalkylrest mit 5-6 C-Atomen,

$R^4$ in Formel I Wasserstoff, Alkyl mit 1-8 C-Atomen, ein Natrium- oder Kaliumkation oder ein Ammoniumion, bedeuten, herstellt.

**4.** Verfahren gemäß Anspruch 1, dadurch gekennzeichnet, daß man Verbindungen der Formel I herstellt.

**5.** Verfahren gemäß Anspruch 1, dadurch gekennzeichnet, daß man die Zwischenstufe der Formel IV

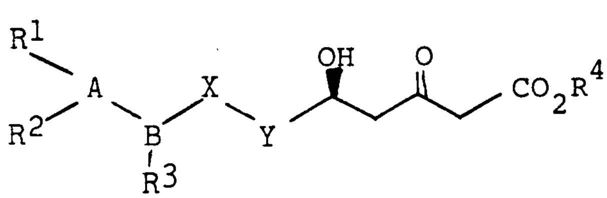

IV

worin A-B, X-Y, $R^1$, $R^2$ und $R^3$ die in Anspruch 1 zu Formeln I und II angegebenen Bedeutung haben und $R^4$ Alkyl mit 1-8 C-Atomen ist, isoliert.

**Claims**

**Claims for the following Contracting States : AT, BE, CH, DE, FR, GB, IT, LI, LU, NL, SE**

**1.** A 3,5-dihydroxycarboxylic acid and its derivatives of the formula I

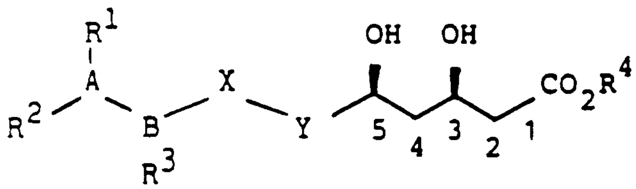

I

and the corresponding lactone of the formula II

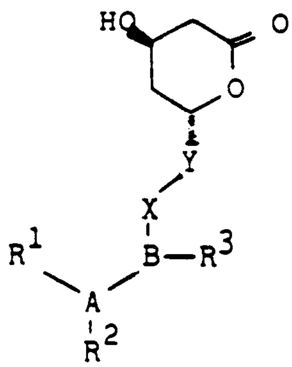

II

where in the formulae I and II

A-B denotes CH-CH or C = C,
X-Y denotes $CH_2$-$CH_2$ or HC = CH,

$R^1$ and $R^2$, $R^1$ and $R^2$ being identical or different, denote a saturated or unsaturated alkyl radical containing up to 20 carbon atoms which may be substituted with an alkoxy group containing 1-6 carbon atoms or the group

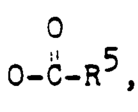

$R^5$ denoting alkyl containing 1-8 carbon atoms, a cycloalkyl radical containing 3-7 carbon atoms, a phenyl, thiophene, furan or naphthalene radical, it being possible for the aromatic radicals to be substituted 1-3 times in the nucleus with halogen, alkyl or alkoxy containing 1-6 carbon atoms in each case, cycloalkyl containing 3-7 carbon atoms or the group

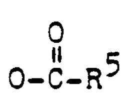

$R^5$ denoting alkyl containing 1-8 carbon atoms, or a pyridine radical which may be substituted 1-3 times in the nucleus with alkyl containing 1-4 carbon atoms,

$R^3$ denotes a saturated or unsaturated alkyl radical containing up to 8 carbon atoms, a benzyl radical which may be substituted 1-3 times in the nucleus with halogen, alkoxy or alkyl containing 1-4 carbon atoms in each case, a phenyl, thiophene, furan, naphthalene radical, it being possible for the aromatic radicals to be substituted 1-3 times in the nucleus with halogen, alkoxy or alkyl containing 1-4 carbon atoms in each case, or a cycloalkyl radical containing 3-7 carbon atoms,

$R^4$ in formulae I denotes hydrogen, alkyl containing 1-8 carbon atoms, a metal cation, an ammonium ion, phenyl, benzyl or the 2,3-dihydroxypropyl radical.

**2.** A compound of the formulae I and II as claimed in claim 1, wherein

A-B denotes CH-CH or C = C,
X-Y denotes $CH_2$-$CH_2$ or HC = CH,

$R^1$ and $R^2$, $R^1$ and $R^2$ being identical or different, denote an alkyl radical containing 1-10 carbon atoms which may be substituted with an alkoxy group containing 1-6 carbon atoms or the group

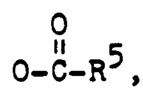

$R^5$ denoting alkyl containing 1-8 carbon atoms, a cycloalkyl radical containing 5-6 carbon atoms, a phenyl radical which may be substituted 1-3 times in the nucleus with halogen, alkyl or alkoxy containing 1-4 carbon atoms in each case, cycloalkyl containing 5-6 carbon atoms, or the group

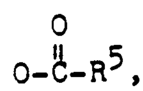

$R^5$ denoting alkyl containing 1-8 carbon atoms,

$R^3$ denotes an alkyl radical containing 1-8 carbon atoms, a benzyl radical which may be substituted 1-3 times in the nucleus with halogen, alkoxy or alkyl containing 1-4 carbon atoms in each case, a phenyl radical which may be substituted 1-3 times in the nucleus with halogen, alkoxy or alkyl containing 1-4 carbon atoms in each case, or a cycloalkyl radical containing 5-6 carbon atoms.

$R^4$ in formula I denotes hydrogen, alkyl containing 1-6 carbon atoms, a metal cation, an ammonium ion, phenyl or benzyl.

**3.** A compound of the formulae I and II as claimed in claim 1, wherein

A-B denotes C = C,
X-Y denotes HC = CH,

$R^1$ and $R^2$, $R^1$ and $R^2$ being identical or different, denote a phenyl radical which may be substituted 1-3 times in the nucleus with halogen, alkyl or alkoxy containing 1 to 4 carbon atoms in each case or the group

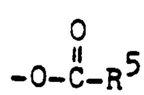

$R^5$ denoting alkyl containing 1-8 carbon atoms,

$R^3$ denotes an alkyl radical containing 1-8 carbon atoms or a cycloalkyl radical containing 5-6 carbon atoms,

$R^4$ in formula I denotes hydrogen, alkyl containing 1-8 carbon atoms, a sodium or potassium cation or an ammonium ion.

**4.** A compound as claimed in claim 1 which has the formula I.

**5.** A process for the preparation of the compounds of the formulae I and II as claimed in claim 1, which comprises

a) converting correspondingly substituted aldehydes of the formula III

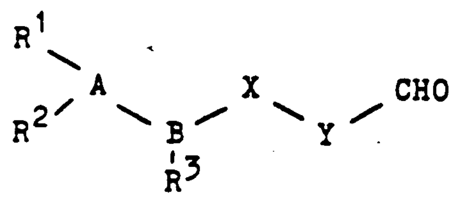

III

in which A-B, X-Y, $R^1$, $R^2$ and $R^3$ have the specified meanings, into the corresponding hydroxyketo esters of the general formula IV

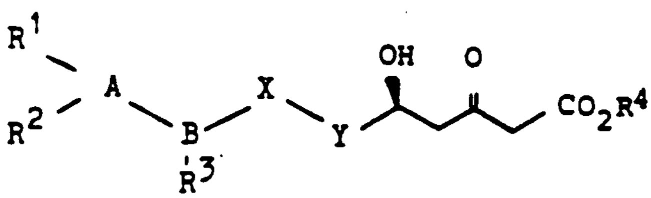

IV

in which A-B, X-Y, $R^1$, $R^2$ and $R^3$ have the specified meanings, and $R^4$ denotes alkyl containing 1-8 carbon atoms,

b) converting the hydroxyketo ester of the formula IV into the corresponding 3,5-dihydroxy compounds of the formula I

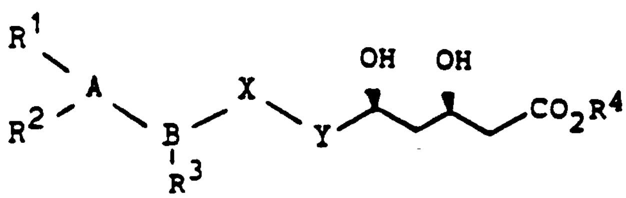

I

in which A-B, X-Y, $R^1$, $R^2$ and $R^3$ have the specified meanings for formula I and $R^4$ is alkyl containing 1-8 carbon atoms and optionally saponifying a compound obtained to a compound of the formula I in which $R^4$ represents a metal cation, optionally the free acid ($R^4$ = hydrogen) being liberated therefrom and the free acid optionally being converted into compounds of the formula I having $R^4$ = ammonium ion, phenyl, benzyl or 2,3-dihydroxypropyl,

c) and a compound of the formula I obtained optionally being converted into a lactone of the formula II

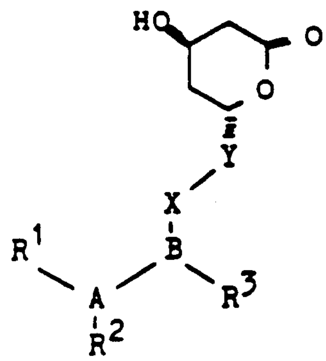

II

in which A-B, X-Y, $R^1$, $R^2$ and $R^3$ have the specified meanings.

**6.** A pharmaceutical product containing a compound as claimed in claim 1.

**7.** A compound as claimed in claim 1 for the prophylaxis and treatment of hypercholesterinemia.

**8.** A compound of the formula IV

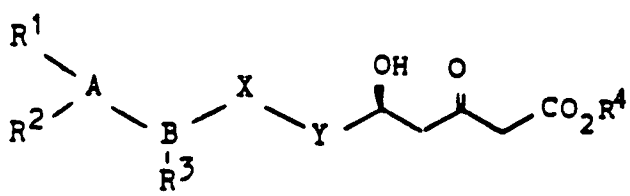

in which A-B, X-Y, $R^1$, $R^2$ and $R^3$ have the meaning specified in claim 1 for formula I and $R^4$ is alkyl containing 1-8 carbon atoms.

**Claims for the following Contracting States : ES, GR**

**1.** A process for the preparation of a 3,5-dihydroxycarboxylic acid and its derivatives of the formula I

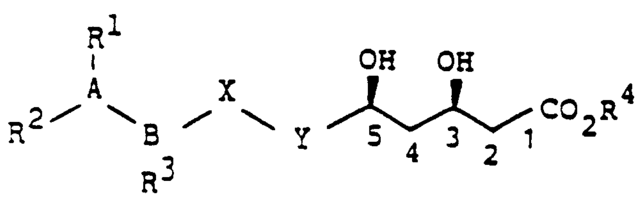

I

and the corresponding lactone of the formula II

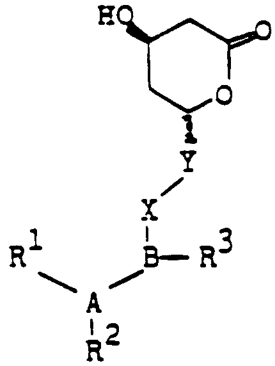

where in the formulae I and II

A-B denotes CH-CH or C = C,
X-Y denotes $CH_2$-$CH_2$ or HC = CH,

$R^1$ and $R^2$, $R^1$ and $R^2$ being identical or different, denote a saturated or unsaturated alkyl radical containing up to 20 carbon atoms which may be substituted with an alkoxy group containing 1-6 carbon atoms or the group

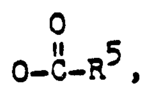

$R^5$ denoting alkyl containing 1-8 carbon atoms, a cycloalkyl radical containing 3-7 carbon atoms, a phenyl, thiophene, furan or naphthalene radical, it being possible for the aromatic radicals to be substituted 1-3 times in the nucleus with halogen, alkyl or alkoxy containing 1-6 carbon atoms in each case, cycloalkyl containing 3-7 carbon atoms or the group

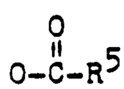

$R^5$ denoting alkyl containing 1-8 carbon atoms, or a pyridine radical which may be substituted 1-3 times in the nucleus with alkyl containing 1-4 carbon atoms,

$R^3$ denotes a saturated or unsaturated alkyl radical containing up to 8 carbon atoms, a benzyl radical which may be substituted 1-3 times in the nucleus with halogen, alkoxy or alkyl containing 1-4 carbon atoms in each case, a phenyl, thiophene, furan, naphthalene radical, it being possible for the aromatic radicals to be substituted 1-3 times in the nucleus with halogen, alkoxy or alkyl containing 1-4 carbon atoms in each case, or a cycloalkyl radical containing 3-7 carbon atoms,

$R^4$ in formula I denotes hydrogen, alkyl containing 1-8 carbon atoms, a metal cation, an ammonium ion, phenyl, benzyl or the 2,3-dihydroxypropyl radical, which comprises

a) converting correspondingly substituted aldehydes of the formula III

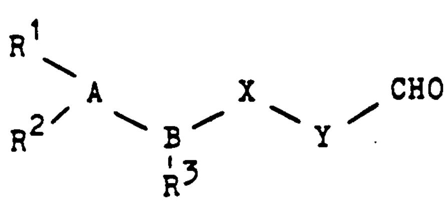

III

in which A-B, X-Y, $R^1$, $R^2$ and $R^3$ have the specified meanings, into the corresponding hydroxyketo esters of the general formula IV

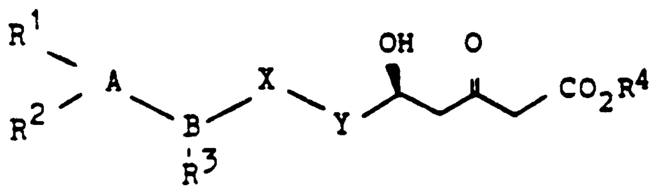

IV

in which A-B, X-Y, $R^1$, $R^2$ and $R^3$ have the specified meanings, and $R^4$ denotes alkyl containing 1-8 carbon atoms,

b) converting the hydroxyketo ester of the formula IV into the corresponding 3,5-dihydroxy compounds of the formula I

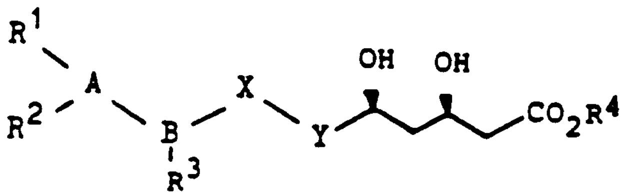

in which A-B, X-Y, $R^1$, $R^2$ and $R^3$ have the specified meanings for formula I and $R^4$ is alkyl containing 1-8 carbon atoms and optionally saponifying a compound obtained to a compound of the formula I in which $R^4$ represents a metal cation, optionally the free acid ($R^4$ = hydrogen) being liberated therefrom and the free acid optionally being converted into compounds of the formula I having $R^4$ = ammonium ion, phenyl, benzyl or 2,3-dihydroxypropyl,

c) and a compound of the formula I obtained optionally being converted into a lactone of the formula II

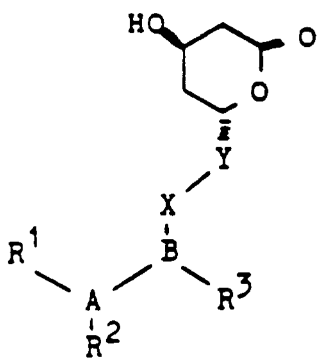

II

in which A-B, X-Y, $R^1$, $R^2$ and $R^3$ have the specified meanings.

**2.** A process as claimed in claim 1, wherein a compound of the formula I or II is prepared, in which

A-B denotes CH-CH or C=C,
X-Y denotes $CH_2$-$CH_2$ or HC = CH,

$R^1$ and $R^2$, $R^1$ and $R^2$ being identical or different, denote an alkyl radical containing 1-10 carbon atoms which may be substituted with an alkoxy group containing 1-6 carbon atoms or the group

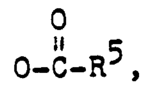

$R^5$ denoting alkyl containing 1-8 carbon atoms, a cycloalkyl radical containing 5-6 carbon atoms, a phenyl radical which may be substituted 1-3 times in the nucleus with halogen, alkyl or alkoxy containing 1-4 carbon atoms in each case, cycloalkyl containing 5-6 carbon atoms, or the group

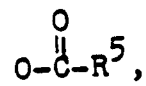

$R^5$ denoting alkyl containing 1-8 carbon atoms,

$R^3$ denotes an alkyl radical containing 1-8 carbon atoms, a benzyl radical which may be substituted 1-3 times in the nucleus with halogen, alkoxy or alkyl containing 1-4 carbon atoms in each case, a phenyl radical which may be substituted 1-3 times in the nucleus with halogen, alkoxy or alkyl containing 1-4 carbon atoms in each case, or a cycloalkyl radical containing 5-6 carbon atoms.

$R^4$ in formula I denotes hydrogen, alkyl containing 1-6 carbon atoms, a metal cation, an ammonium ion, phenyl or benzyl.

**3.** A process as claimed in claim 1, wherein a compound of the formula I or II is prepared, in which

A-B denotes C = C,
X-Y denotes HC = CH,

$R^1$ and $R^2$, $R^1$ and $R^2$ being identical or different, denote a phenyl radical which may be substituted 1-3 times in the nucleus with halogen, alkyl or alkoxy containing 1 to 4 carbon atoms in each case or the group

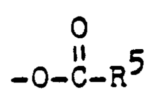

$R^5$ denoting alkyl containing 1-8 carbon atoms,

$R^3$ denotes an alkyl radical containing 1-8 carbon atoms or a cycloalkyl radical containing 5-6 carbon atoms,

$R^4$ in formula I denotes hydrogen, alkyl containing 1-8 carbon atoms, a sodium or potassium cation or an ammonium ion.

**4.** A process as claimed in claim 1, wherein a compound of the formula I is prepared.

**5.** A process as claimed in claim 1, wherein an intermediate of the formula IV

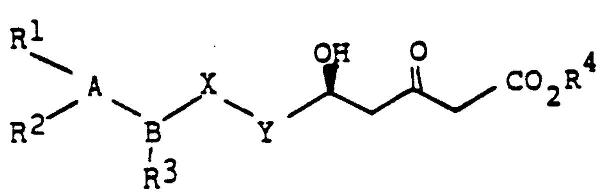

IV

in which A-B, X-Y, $R^1$, $R^2$ and $R^3$ have the meaning specified in claim 1 for formulae I and II and $R^4$ is alkyl containing 1-8 carbon atoms, is isolated.

**Revendications**

**Revendications pour les Etats contractants suivants : AT, BE, CH, DE, FR, GB, IT, LI, LU, NL, SE**

**1.** Acides 3,5-dihydroxycarboxyliques et leurs dérivés de formule générale I

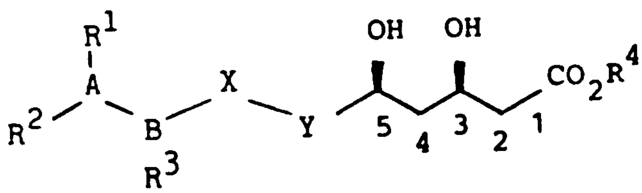

I

et les lactones correspondantes de formule II

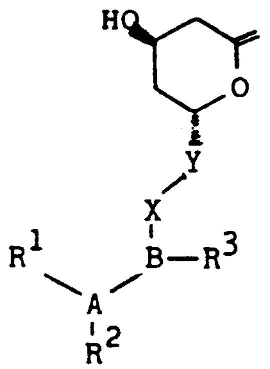

II

dans les formules générales I et II,

A-B représentant CH-CH ou C = C,
X-Y représentant $CH_2$-$CH_2$ ou HC = CH,

$R^1$ et $R^2$, $R^1$ et $R^2$ étant identiques ou différents, représentant un radical alkyle saturé ou insaturé ayant jusqu'à 20 atomes de carbone, qui peut être substitué par un groupe alcoxy ayant de 1 à 6 atomes de carbone ou par le groupe

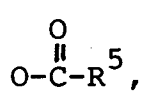

$R^5$ représentant un groupe alkyle ayant de 1 à 8 atomes de carbone, un radical cycloalkyle ayant de 3 à 7 atomes de carbone, un radical phényle, thiophène, furanne ou naphtalène, les radicaux aromatiques pouvant être 1-3 fois substitués sur le noyau par un ou des atomes d'halogène ou groupes alkyle ou alcoxy ayant chacun de 1 à 6 atomes de carbone, cycloalkyle ayant de 3 à 7 atomes de carbone ou le groupe

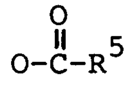

$R^5$ représentant un groupe alkyle ayant de 1 à 8 atomes de carbone, ou un radical pyridino qui peut

être 1-3 fois substitué sur le noyau par des groupes alkyle ayant de 1 à 4 atomes de carbone;
$R^3$ représentant un radical alkyle saturé ou insaturé ayant jusqu'à 8 atomes de carbone, un radical benzyle qui peut être-1-3 fois substitué sur le noyau par un ou des atomes d'halogène ou groupes alcoxy ou alkyle ayant chacun de 1 à 4 atomes de carbone, un radical phényle, thiophène, furanne, naphtalène, les radicaux aromatiques pouvant être 1-3 fois substitués sur le noyau par un ou des atomes d'halogène ou groupes alcoxy ou alkyle ayant chacun 1-4 atomes de carbone, ou un radical cycloalkyle ayant 3-7 atomes de carbone.
$R^4$ dans la formule I représentant un atome d'hydrogène ou un groupe alkyle ayant 1-8 atomes de carbone, un cation métallique, un ion ammonium, le radical phényle, benzyle ou 2,3-dihydroxypropyle.

**2.** Composés de formules générales I et II selon la revendication 1, caractérisés en ce que

A-B représente CH-CH ou C = C,
X-Y représente $CH_2$-$CH_2$ ou HC = CH,

$R^1$ et $R^2$, $R^1$ et $R^2$ étant identiques ou différents, représentent un radical alkyle ayant de 1 à 10 atomes de carbone, qui peut être substitué par un groupe alcoxy ayant de 1 à 6 atomes de carbone ou par le groupe

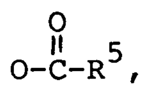

$R^5$ représentant un groupe alkyle ayant de 1 à 8 atomes de carbone, un radical cycloalkyle ayant 5-6 atomes de carbone, un radical phényle qui peut être 1-3 fois substitué sur le noyau par un ou des atomes d'halogène ou groupes alkyle ou alcoxy ayant chacun de 1 à 4 atomes de carbone, cycloalkyle ayant 5 ou 6 atomes de carbone ou le groupe

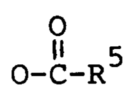

$R^5$ représentant un groupe alkyle ayant de 1 à 8 atomes de carbone;
$R^3$ représente un radical alkyle ayant de 1 à 8 atomes de carbone, un radical benzyle qui peut être 1-3 fois substitué sur le noyau par un ou des atomes d'halogène ou groupes alcoxy ou alkyle ayant chacun de 1 à 4 atomes de carbone, un radical phényle qui peut être 1-3 fois substitué sur le noyau par un ou des atomes d'halogène ou groupes alcoxy ou alkyle ayant chacun de 1 à 4 atomes de carbone, ou un radical cycloalkyle ayant 5 ou 6 atomes de carbone;
$R^4$ dans la formule I représente un atome d'hydrogène ou un groupe alkyle ayant de 1 à 6 atomes de carbone, un cation métallique, un ion ammonium, le groupe phényle ou benzyle.

**3.** Composés de formules générales I et II selon la revendication 1, caractérisés en ce que

A-B représente C = C,
X-Y représente HC = CH,

$R^1$ et $R^2$, $R^1$ et $R^2$ étant identiques ou différents, représentent un radical phényle qui peut être 1-3 fois substitué sur le noyau par un ou des atomes d'halogène ou groupes alkyle ou alcoxy ayant chacun de 1 à 4 atomes de carbone ou par le groupe

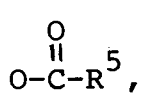

$R^5$ représentant un groupe alkyle ayant de 1 à 8 atomes de carbone;

$R^3$ représente un radical alkyle ayant de 1 à 8 atomes de carbone ou un radical cycloalkyle ayant 5-6 atomes de carbone;
$R^4$ dans la formule I représente un atome d'hydrogène ou un groupe alkyle ayant de 1 à 8 atomes de carbone, un cation sodium ou potassium ou un ion ammonium.

**4.** Composés selon la revendication 1, caractérisés en ce qu'ils possèdent la formule I.

**5.** Procédé pour la préparation des composés de formules générales I et II selon la revendication 1, caractérisé en ce que

a) on convertit des aldéhydes convenablement substitués de formule III

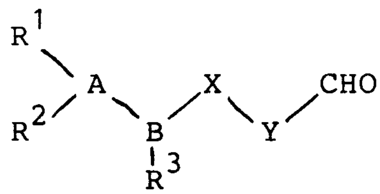

III

dans laquelle A-B, X-Y $R^1$, $R^2$ et $R^3$ ont les significations indiquées, en les hydroxycétoesters correspondants de formule générale IV

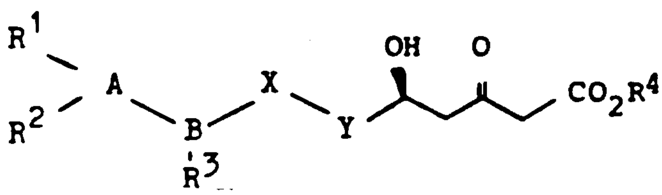

IV

dans laquelle A-B, X-Y, $R^1$, $R^2$ et $R^3$ ont les significations indiquées et $R^4$ représente un groupe alkyle ayant de 1 à 8 atomes de carbone;

b) on convertit les hydroxycétoesters de formule IV en les composés 3,5-dihydroxy correspondants de formule I

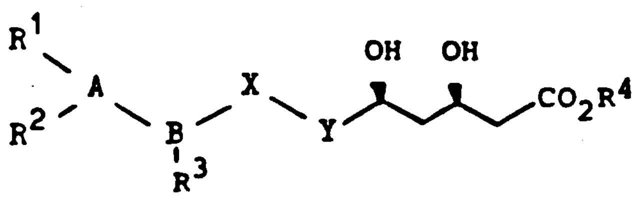

I

dans laquelle A-B, X-Y, $R^1$, $R^2$ et $R^3$ ont les significations données à propos de la formule I, et $R^4$ est un groupe alkyle ayant de 1 à 8 atomes de carbone, et éventuellement on saponifie un composé obtenu en un composé de formule I dans lequel $R^4$ représente un cation métallique, éventuellement on libère à partir de celui-ci l'acide libre ($R^4$ = H) et éventuellement on convertit l'acide libre en composés de formule I dans lesquels $R^4$ représente l'ion ammonium ou le groupe phényle, benzyle ou 2,3-dihydroxypropyle;

c) et on convertit éventuellement un composé de formule I obtenu en une lactone de formule II

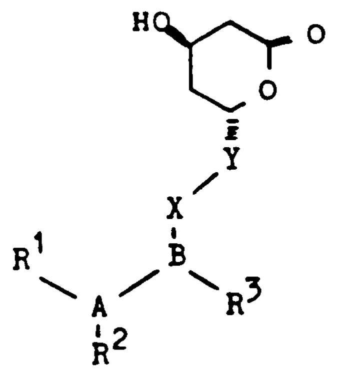

II

dans laquelle A-B, X-Y, $R^1$, $R^2$ et $R^3$ ont les significations indiquées.

**6.** Compositions pharmaceutiques contenant un composé selon la revendication 1.

**7.** Composé selon la revendication 1, pour la prophylaxie et le traitement de l'hypercholestérolémie.

**8.** Composés de formule IV

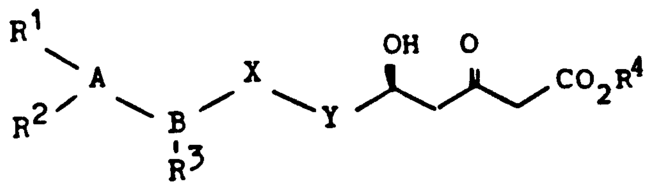

dans laquelle A-B, X-Y, $R^1$, $R^2$ et $R^3$ ont les significations indiquées à propos de la formule I dans la revendication 1, et $R^4$ est un groupe alkyle ayant de 1 à 8 atomes de carbone.

**Revendications pour les Etats contractants suivants : ES, GR**

**1.** Procédé pour la préparation d'acides 3,5-dihydroxycarboxyliques et de leurs dérivés de formule générale I

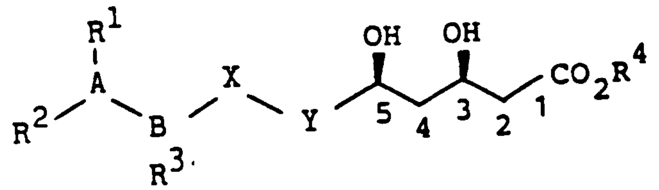

I

et des lactones correspondantes de formule II

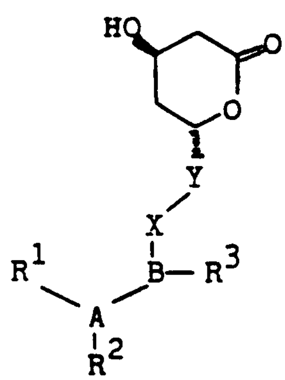

II

dans les formules générales I et II,

A-B représentant CH-CH ou C = C,
X-Y représentant $CH_2$-$CH_2$ ou HC = CH,

$R^1$ et $R^2$, $R^1$ et $R^2$ étant identiques ou différents, représentant un radical alkyle saturé ou insaturé ayant jusqu'à 20 atomes de carbone, qui peut être substitué par un groupe alcoxy ayant de 1 à 6 atomes de carbone ou par le groupe

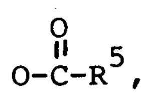

$R^5$ représentant un groupe alkyle ayant de 1 à 8 atomes de carbone, un radical cycloalkyle ayant de 3 à 7 atomes de carbone, un radical phényle, thiophène, furanne ou naphtalène, les radicaux aromatiques pouvant être 1-3 fois substitués sur le noyau par un ou des atomes d'halogène ou groupes alkyle ou alcoxy ayant chacun de 1 à 6 atomes de carbone, cycloalkyle ayant de 3 à 7 atomes de carbone ou le groupe

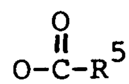

$R^5$ représentant un groupe alkyle ayant de 1 à 8 atomes de carbone, ou un radical pyridino qui peut être 1-3 fois substitué sur le noyau par des groupes alkyle ayant de 1 à 4 atomes de carbone;
$R^3$ représentant un radical alkyle saturé ou insaturé ayant jusqu'à 8 atomes de carbone, un radical benzyle qui peut être 1-3 fois substitué sur le noyau par un ou des atomes d'halogène ou groupes alcoxy ou alkyle ayant chacun de 1 à 4 atomes de carbone, un radical phényle, thiophène, furanne, naphtalène, les radicaux aromatiques pouvant être 1-3 fois substitués sur le noyau par un ou des atomes d'halogène ou groupes alcoxy ou alkyle ayant chacun 1-4 atomes de carbone, ou un radical cycloalkyle ayant 3-7 atomes de carbone.
$R^4$ dans la formule I, représentant un atome d'hydrogène ou un groupe alkyle ayant 1-8 atomes de carbone, un cation métallique, un ion ammonium, le radical phényle, benzyle ou 2,3-dihydroxypropyle,
caractérisé en ce que

a) on convertit des aldéhydes convenablement substitués de formule III

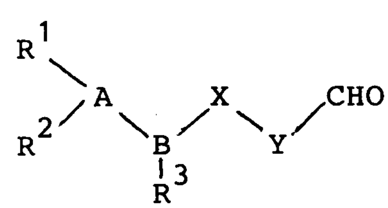

III

dans laquelle A-B, X-Y $R^1$, $R^2$ et $R^3$ ont les significations indiquées, en les hydroxycétoesters correspondants de formule générale IV

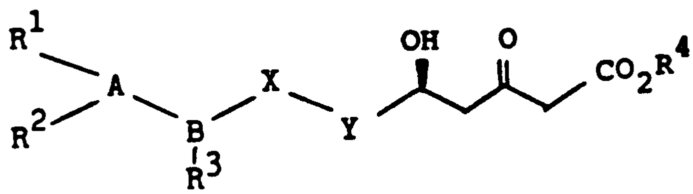

IV

dans laquelle A-B, X-Y, $R^1$, $R^2$ et $R^3$ ont les significations indiquées et $R^4$ représente un groupe alkyle ayant de 1 à 8 atomes de carbone;

b) on convertit les hydroxycétoesters de formule IV en les composés 3,5-dihydroxy correspondants de formule I

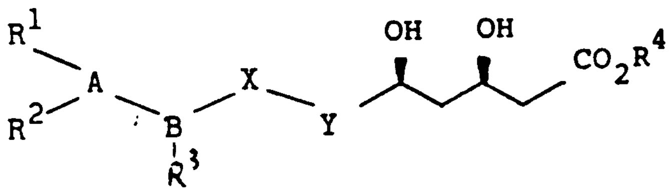

I

dans laquelle A-B, X-Y, $R^1$, $R^2$ et $R^3$ ont les significations données à propos de la formule I, et $R^4$ est un groupe alkyle ayant de 1 à 8 atomes de carbone, et éventuellement on saponifie un composé obtenu en un composé de formule I dans lequel $R^4$ représente un cation métallique, éventuellement on libère à partir de celui-ci l'acide libre ($R^4$ = H) et éventuellement on convertit l'acide libre en composés de formule I dans lesquels $R^4$ représente l'ion ammonium ou le groupe phényle, benzyle ou 2,3-dihydroxypropyle;

c) et on convertit éventuellement un composé de formule I obtenu en une lactone de formule II

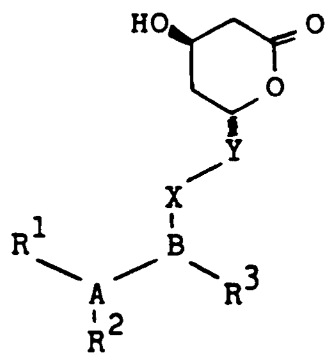

II

dans laquelle A-B, X-Y, $R^1$, $R^2$ et $R^3$ ont les significations indiquées.

**2.** Procédé selon la revendication 1, caractérisé en ce que l'on prépare un composé de formule générale I ou II, dans lequel

A-B représente CH-CH ou C = C,
X-Y représente $CH_2$-$CH_2$ ou HC = CH,

$R^1$ et $R^2$, $R^1$ et $R^1$ étant identiques ou différents, représentent un radical alkyle ayant de 1 à 10 atomes de carbone, qui peut être substitué par un groupe alcoxy ayant de 1 à 6 atomes de carbone ou par le groupe

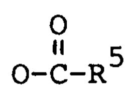,

$R^5$ représentant un groupe alkyle ayant de 1 à 8 atomes de carbone, un radical cycloalkyle ayant 5-6 atomes de carbone, un radical phényle qui peut être 1-3 fois substitué sur le noyau par un ou des atomes d'halogène ou groupes alkyle ou alcoxy ayant chacun de 1 à 4 atomes de carbone, cycloalkyle ayant 5 ou 6 atomes de carbone ou le groupe

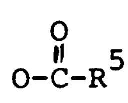

$R^5$ représentant un groupe alkyle ayant de 1 à 8 atomes de carbone;
$R^3$ représente un radical alkyle ayant de 1 à 8 atomes de carbone, un radical benzyle qui peut être 1-3

fois substitué sur le noyau par un ou des atomes d'halogène ou groupes alcoxy ou alkyle ayant chacun de 1 à 4 atomes de carbone, un radical phényle qui peut être 1-3 fois substitué sur le noyau par un ou des atomes d'halogène ou groupes alcoxy ou alkyle ayant chacun de 1 à 4 atomes de carbone, ou un radical cycloalkyle ayant 5 ou 6 atomes de carbone;
$R^4$ dans la formule I représente un atome d'hydrogène ou un groupe alkyle ayant de 1 à 6 atomes de carbone, un cation métallique, un ion ammonium, le groupe phényle ou benzyle.

**3.** Procédé selon la revendication 1, caractérisé en ce que l'on prépare un composé de formule générale I ou II, dans lequel

A-B représente C=C,
X-Y représente HC=CH,

$R^1$ et $R^2$, $R^1$ et $R^2$ étant identiques ou différents, représentent un radical phényle qui peut être 1-3 fois substitué sur le noyau par un ou des atomes d'halogène ou groupes alkyle ou alcoxy ayant chacun de 1 à 4 atomes de carbone ou par le groupe

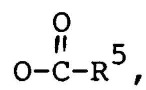

$R^5$ représentant un groupe alkyle ayant de 1 à 8 atomes de carbone;
$R^3$ représente un radical alkyle ayant de 1 à 8 atomes de carbone ou un radical cycloalkyle ayant 5-6 atomes de carbone;
$R^4$ dans la formule I représente un atome d'hydrogène ou un groupe alkyle ayant de 1 à 8 atomes de carbone, un cation sodium ou potassium ou un ion ammonium.

**4.** Procédé selon la revendication 1, caractérisé en ce que l'on prépare des composés de formule I.

**5.** Procédé selon la revendication 1, caractérisé en ce que l'on isole les produits intermédiares de formule IV

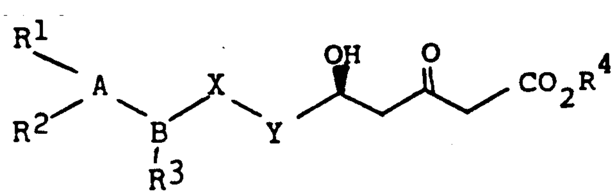

IV

dans laquelle A-B, X-Y, $R^1$, $R^2$ et $R^3$ ont les significations données à propos des formules I et II dans la revendication 1, et $R^4$ est un groupe alkyle ayant de 1 à 8 atomes de carbone.